# EUROPEAN PATENT APPLICATION

(11) **EP 4 364 657 A1**
(43) Date of publication of application: **08.05.2024**
(21) Application number: 22853250.3
(22) Date of filing: 02.06.2022
(51) Int. Cl.: A61B 5/145, A61B 5/15, A61B 5/151, A61B 5/155, A61B 5/00

(54) **APPLICATOR FOR TRANSCUTANEOUS SENSOR AND APPLICATOR ASSEMBLY**

(30) Priority: 05.08.2021 KR 20210103243
(71) Applicant: i-Sens, Inc., Seoul 06646 (KR)
(72) Inventor: CHOI, Hyun Ho, Seoul 06646 (KR); RYU, Goang Yel, Seoul 06646 (KR); WANG, Ji Hoon, Seoul 06646 (KR)
(74) Representative: BCKIP Part mbB
(86) International application number: PCT/KR2022/007870
(87) International publication number: WO 2023/013870

(57) **Abstract**

An applicator for inserting a sensor measuring biometric information into the skin of a user according to the present disclosure, comprising: an applicator body having an applicator body coupling portion; an insertion unit for moving a sensor unit from a first position spaced apart from the user's skin to a second position where the sensor is inserted into the user's skin, the sensor unit including the sensor and a sensor unit housing in which the sensor is attached; and a column member which has the insertion unit attached thereto, comprises a column member coupling portion corresponding to the applicator body coupling portion, and is fixed to the applicator body in a manner that the column member coupling portion is engaged with the applicator body coupling portion.

## Description

### TECHNICAL FIELD

The present disclosure relates to an applicator for a transcutaneous sensor, and more specifically, related to an applicator and an applicator assembly for inserting a transcutaneous sensor, which is inserted into the skin of a user to measure biometric information, into the skin of a user.

### BACKGROUND

With the recent advancement of medical technology, various medical devices that are attached to the body of a user have been developed and sold. Medical devices that are attached to the skin can be useful for monitoring biometric information or providing treatment by attaching them to the body of a patient with a chronic disease.

For example, chronic diseases such as diabetes require continuous management, and a body attachable unit for measuring biometric information can be used to manage blood glucose levels in diabetic patients. Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown. Further progression of diabetes leads to complications such as visual disturbances, hypertension, kidney disease, paralysis, periodontal disease, muscle spasms and neuralgia, as well as gangrene. In order to diagnose diabetes beforehand and manage to prevent the progression of diabetes into complications associated therewith, systematic blood glucose measurement and treatment should be performed.

For diabetes patients as well as people having higher than normal blood glucose, even though diabetes has not yet developed, medical device manufacturers offer a variety of blood glucose meters to measure blood glucose levels.

Glucose measuring devices may be categorized into a single time measurement type measuring a blood glucose level and collecting blood from a fingertip by a user every single time and a continuous measurement type attaching a glucose monitoring system to the belly or an arm of the user and continuously measuring blood glucose levels.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

Recently, to overcome such a drawback, continuous glucose monitoring systems (CGMSs) inserted into the human body to measure a blood glucose level every few minutes have been developed, and therefore easily perform the management of diabetics and responses to an emergency situation.

A continuous blood glucose measurement system includes a body attachable unit that includes a transcutaneous sensor that is inserted into the skin of a user and measures blood glucose levels in the body, and a terminal that receives biometric information transmitted from the body attachable unit and outputs.

Systems for medical purposes using transcutaneous sensors are produced in many different forms by each manufacturer, and the methods of use also vary. Most systems currently manufactured and distributed are a method of attaching a disposable sensor unit to a body by an applicator. A user needs to perform several steps to attach the sensor unit to a skin using an applicator, and after attaching the sensor unit to a body, various follow-up procedures such as having to directly pull out the needle inserted into the skin along with the transcutaneous sensor are required to be performed.

For example, a user must peel off the packaging of the disposable sensor unit and accurately attach it to the applicator, and then insert the sensor unit into the applicator and operate the applicator to attach the sensor unit to a skin. Additionally, after attaching the sensor unit, there is the inconvenience of having to perform tasks such as pulling out the needle inserted into the skin and coupling the transmitter to the sensor unit.

In addition, conventional applicators have problems in that assembly of parts is cumbersome and manufacturing time is long.

### SUMMARY

### Technical Problem

The present disclosure is developed in consideration of the above-described points, and the purpose of the present disclosure is to provide an applicator and applicator assembly that facilitates assembly of parts and reduces manufacturing time by using a method of making important parts into one assembly and combining it with other parts.

### Solution to Problem

To accomplish the purposes described above, according to an embodiment of the present disclosure, an applicator for inserting a sensor for measuring biometric information into skin of a user may comprise: an applicator body having an applicator body coupling portion; an insertion unit to move a sensor unit, including the sensor and a sensor unit housing to which the sensor is mounted, from a first position spaced apart from the skin of the user to a second position where the sensor is inserted into the skin of the user; and a column member which the insertion unit is mounted to, has a column member coupling portion corresponding to the applicator body coupling portion, and is fixed to the applicator body in a way that the column member coupling portion is engaged with the applicator body coupling portion.

The applicator body coupling portion may include an applicator body groove formed at one side of the applicator body, the column member may include a column member body having a space, in which the insertion unit is mounted, inside the column member body, and the column member coupling portion may include a coupling end portion provided at one end of the column member body to be inserted into the applicator body groove.

The applicator body coupling portion may include a fence portion configured to be elastically deformable in the applicator body groove to support the coupling end portion by being contacted with an outer surface of the coupling end portion.

The coupling end portion may include a plurality of column member legs configured to be elastically deformable at one end of the column member body to contactable with the applicator body.

One of the applicator body coupling portion and the column member coupling portion may have an elastically deformable snap hook, and another of the applicator body coupling portion and the column member coupling portion has a snap groove into which a portion of the snap hook is inserted.

The insertion unit may comprise a shuttle configured to be movable from the first position to the second position together with the sensor unit, and an elastic member configured to apply an elastic force to the shuttle in a direction of moving toward the second position with respect to the shuttle, and the column member may be configured to guide the shuttle to move linearly the shuttle.

The shuttle may be temporarily fixed to the column member such that the column member maintains a position in which the elastic member is elastically deformed by the clamping portion, which connects the shuttle and the column member, before the column member is coupled to the applicator body, and after the column member is coupled to the applicator body, a temporarily fixed state by the clamping portion is released.

The clamping portion may include a clamping hook elastically deformably connected to the shuttle to be engaged with a column member detent provided at one side of the column member, and the shuttle may be configured to maintain a position in which the elastic member is elastically deformed when the clamping hook is engaged with the column member detent, and the temporarily fixed state is released when the clamping hook is disengaged from the column member detent.

The clamping portion may include a clamping portion arm provided to be elastically deformable at the column member to be engaged with a shuttle latch provided at one side of the shuttle, wherein the shuttle is configured to maintain a position in which the elastic member is elastically deformed when the clamping portion arm is engaged with the shuttle latch, and the temporarily fixed state is released when the clamping portion arm is disengaged from the shuttle latch.

The column member may be provided with a blocking portion configured to restrict movement of the shuttle to prevent the shuttle from returning to the first position by being contacted with one side of the shuttle after the shuttle moves to the second position.

The insertion unit may include a shuttle configured to move from the first position to the second position together with the sensor unit, and an elastic member configured to apply elastic force to the shuttle in a direction of moving to the second position with respect to the shuttle, the column member may be configured to guide the shuttle to move linearly the shuttle, and the shuttle may be temporarily fixed to the column member to maintain a position of elastically deforming the elastic member by a safety pin that passes through the column member hole formed at one side of the column member and is inserted into the shuttle hole provided at one side of the shuttle before the column member is coupled to the applicator body, and, after the column member is coupled to the applicator body, a state temporarily fixed by the safety pin is released.

The insertion unit may include a carrier relatively movably mounted to the shuttle and having a carrier hole into which the safety pin is insertable, a needle coupled to the carrier to be inserted into the skin of the user together with the sensor, and a needle release elastic member configured to apply elastic force to the carrier in a direction in which the needle is extracted from the skin of the user, and the carrier may be temporarily fixed to the shuttle to maintain a position in which the needle release elastic member is elastically deformed by inserting the safety pin into the carrier hole before the column member is coupled to the applicator body, and after the column member is coupled to the applicator body, the state temporarily fixed by the safety pin is released.

Additionally, to accomplish the purposes described above, an applicator assembly may comprise: an applicator body having an applicator body coupling portion; a sensor unit including a sensor and a sensor unit housing to which the sensor is mounted; an insertion unit to move the sensor unit from a first position spaced apart from the skin of the user to a second position where the sensor is inserted into the skin of the user; and a column member which the insertion unit is mounted to, has a column member coupling portion corresponding to the applicator body coupling portion, and is fixed to the applicator body in a way that the column member coupling portion is engaged with the applicator body coupling portion.

The applicator assembly according to an embodiment of the present disclosure may comprise a base unit including a base unit housing to which the sensor unit housing is coupled and an adhesive portion provided at the base unit housing to be attached to the skin of the user, the base unit configured to be spaced apart from the sensor unit to be separably coupled to the applicator body, wherein the sensor unit housing is coupled to the base unit housing at the second position.

### Advantageous Effects of Invention

According to the present disclosure, the insertion unit including the shuttle that moves the sensor unit can be simply assembled to the applicator body by forming one assembly with the column member which is coupled to the applicator body. Therefore, the insertion unit can be easily installed, manufacturing time can be reduced, and assembly efficiency can be improved. Additionally, the sensor unit can be simply installed on the applicator body in a state in which the sensor unit is coupled to the insertion unit from the outside of the applicator body.

In addition, according to the present disclosure, the insertion unit can be coupled to the applicator body in a locked state to prevent accidental movement. And, after the insertion unit is coupled to the applicator body, it may be converted to an operable state through simple manipulation. Therefore, after installing the insertion unit into the applicator body, there is no need for cumbersome follow-up work to bring the shuttle of the insertion unit into an operational state.

In addition, according to the present disclosure, by manufacturing the sensor unit in an assembled state within the applicator, additional work of a user to attach the sensor unit to the skin of a user is minimized, and the sensor unit can be attached to the skin of a user simply by operating the applicator.

### BRIEF DESCRIPTION OF THE DRAWINGS

FIG. 1 is a perspective view showing an applicator assembly according to an embodiment of the present disclosure.
FIG. 2 shows a body attachable unit attached to the skin of a user.
FIG. 3 is a perspective view showing a body attachable unit.
FIG. 4 shows a state in which a needle is coupled to a sensor unit.
FIG. 5 shows a sensor unit and a base unit before they are coupled.
FIG. 6 is a cross-sectional view taken along line I-I in FIG. 1.
FIG. 7 is a cross-sectional view taken along line II-II in FIG. 1.
FIG. 8 is an exploded perspective view of the applicator according to an embodiment of the present disclosure.
FIG. 9 shows an exploded view of the insertion unit assembly.
FIG. 10 shows a state in which a carrier and the needle of a needle assembly are separated.
FIGS. 11 and 12 are exploded views of a part of an applicator according to an embodiment of the present disclosure.
FIG. 13 is for explaining how the sensor unit is mounted on the shuttle of the insertion unit.
FIG. 14 shows the column member and insertion unit separated.
FIG. 15 shows a state in which the shuttle and carrier of the insertion unit are temporarily fixed to the column member.
FIG. 16 shows the insertion unit assembly coupled to the applicator body.
FIG. 17 shows the safety pin being removed from the insertion unit assembly.
FIG. 18 shows the process of disengaging the clamping portion of the shuttle from the column member.
FIG. 19 shows the sensor insertion operation of the insertion unit.
FIG. 20 shows the process of moving the stopper member from the third position to the fourth position.
FIGS. 21 to 23 show a process in which the sensor of the sensor unit is inserted into the skin of a user by an applicator according to an embodiment of the present disclosure.
FIG. 24 is a cross-sectional view showing an applicator assembly according to another embodiment of the present disclosure.
FIG. 25 shows the sensor unit of the applicator assembly shown in FIG. 24.
FIG. 26 is an exploded perspective view showing an applicator assembly according to another embodiment of the present disclosure.
FIG. 27 is a perspective view of a part of the applicator assembly shown in FIG. 26.

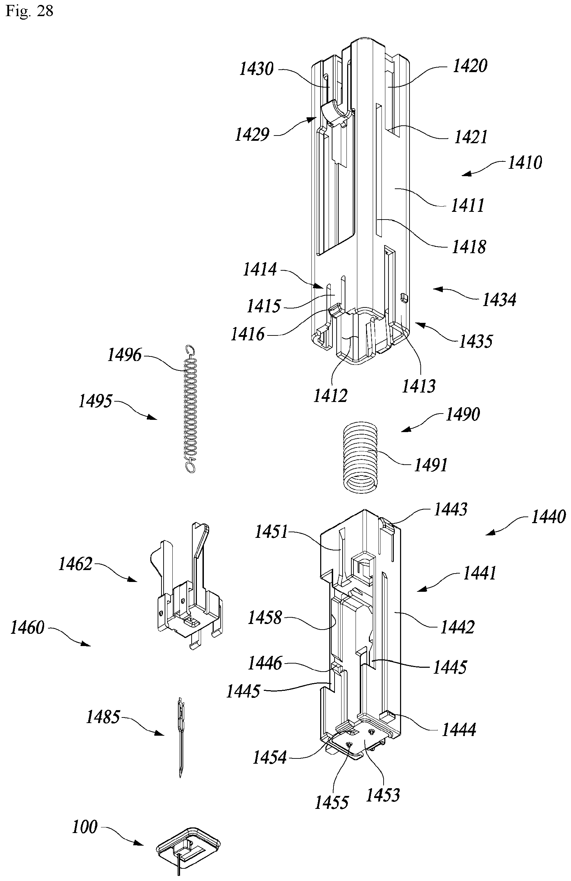

FIG. 28 is an exploded perspective view of the insertion unit assembly of the applicator assembly shown in FIG. 26.

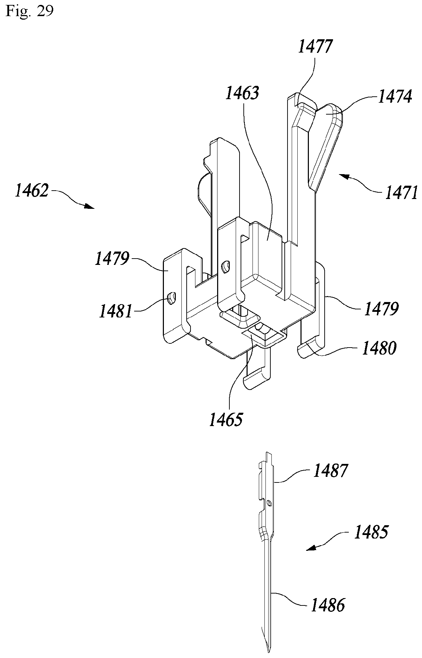

FIG. 29 is an exploded perspective view of the carrier and needle of the applicator assembly shown in FIG. 26.
FIG. 30 is for explaining how the sensor unit is mounted on the shuttle of the applicator assembly shown in FIG. 26.

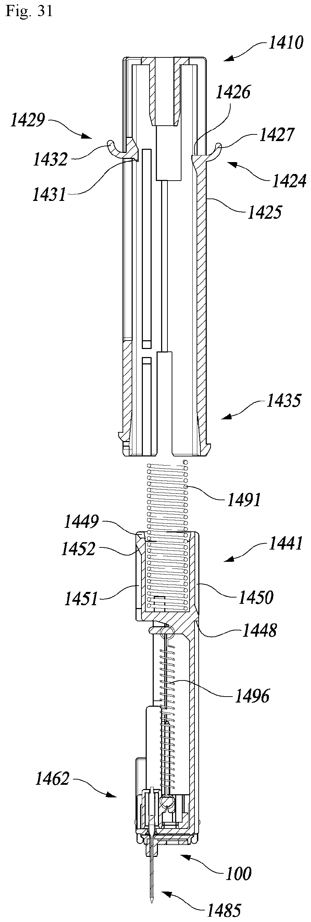

FIG. 31 is a cross-sectional view showing the column member and insertion unit of the applicator assembly shown in FIG. 26 separated.
FIG. 32 is a cross-sectional view showing the column member and insertion unit of the applicator assembly shown in FIG. 26 combined.
FIG. 33 is a cross-sectional view showing the insertion unit assembly of the applicator assembly shown in FIG. 26 coupled to the applicator body.
FIGS. 34 and 35 show a process in which the sensor of the sensor unit is inserted into the skin of a user by the applicator of the applicator assembly shown in FIG. 26.
FIG. 36 is a cross-sectional view showing an applicator assembly according to another embodiment of the present disclosure.
FIGS. 37 and 38 are exploded perspective views of a part of the applicator assembly shown in FIG. 36.

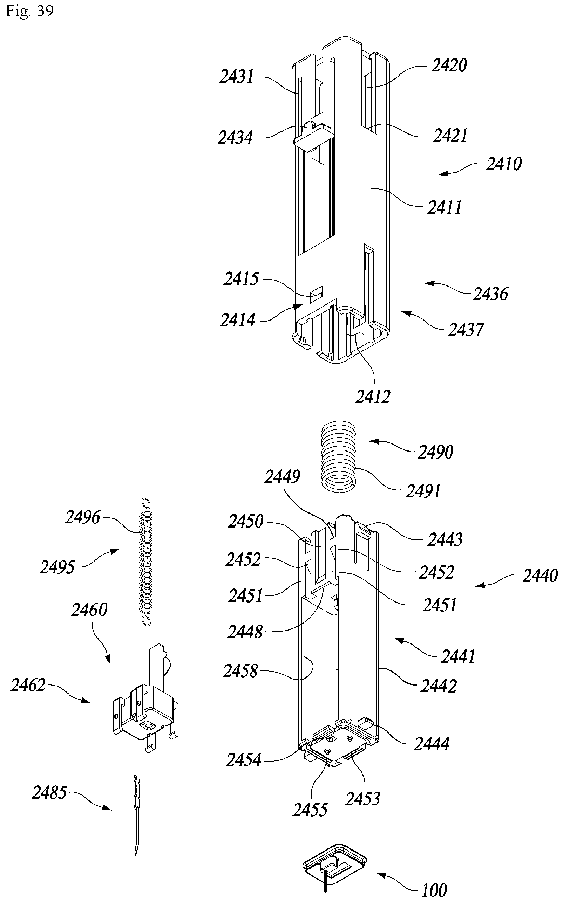

FIG. 39 is an exploded perspective view of the insertion unit assembly of the applicator assembly shown in FIG. 36.

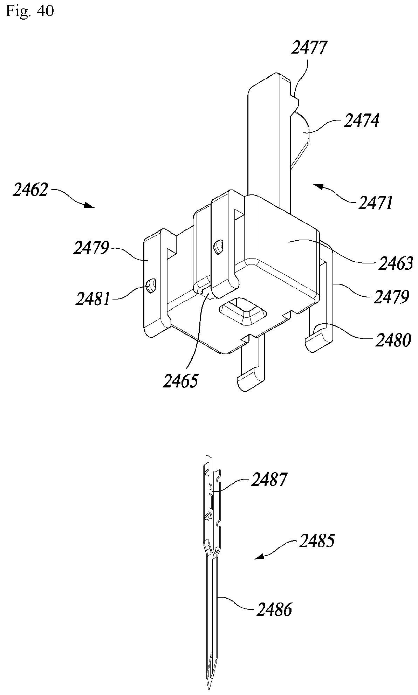

FIG. 40 is an exploded perspective view of the carrier and needle of the applicator assembly shown in FIG. 36.

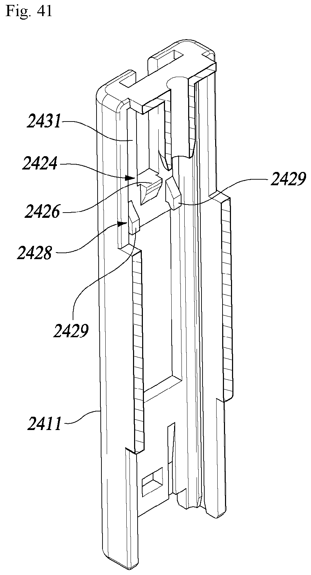

FIG. 41 shows the cutting of the column member of the applicator assembly shown in FIG. 36.

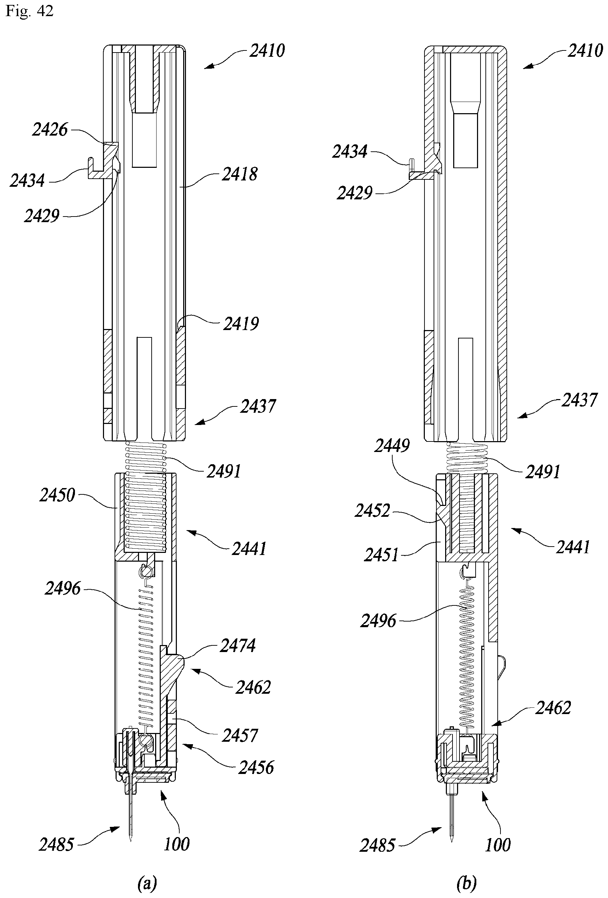

FIG. 42 is a cross-sectional view showing the column member and insertion unit of the applicator assembly shown in FIG. 36 separated.

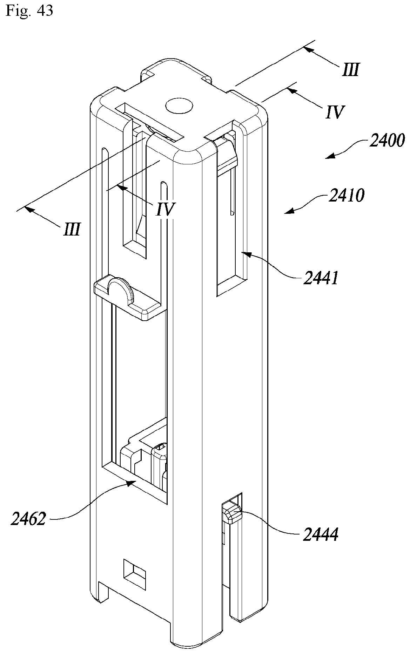

FIG. 43 is a perspective view showing the insertion unit assembly of the applicator assembly shown in FIG. 36.
FIG. 44 is a cross-sectional view taken along line III-III and IV-IV of FIG. 43.
FIG. 45 is a cross-sectional view illustrating a state in which an insertion unit assembly of an applicator assembly shown in FIG. 36 is coupled to an applicator body.
FIGS. 46 and 47 illustrate a process in which a sensor of a sensor unit is inserted into skin of a user by an applicator of an applicator assembly shown in FIG. 36.

### DETAILED DESCRIPTION OF EMBODIMENTS

Hereinafter, the applicator and applicator assembly for a transcutaneous sensor according to the present disclosure will be described in detail with reference to the drawings.

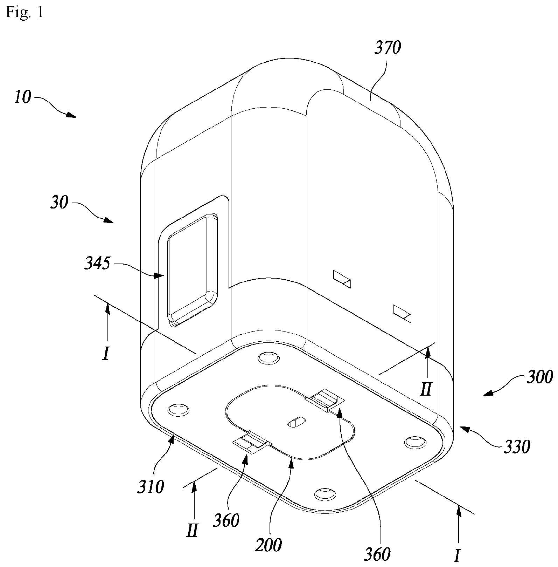

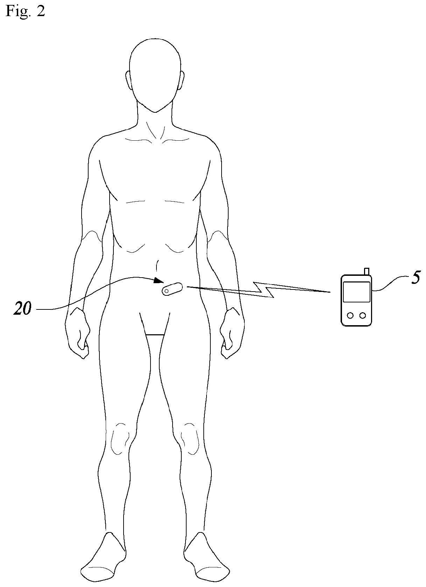

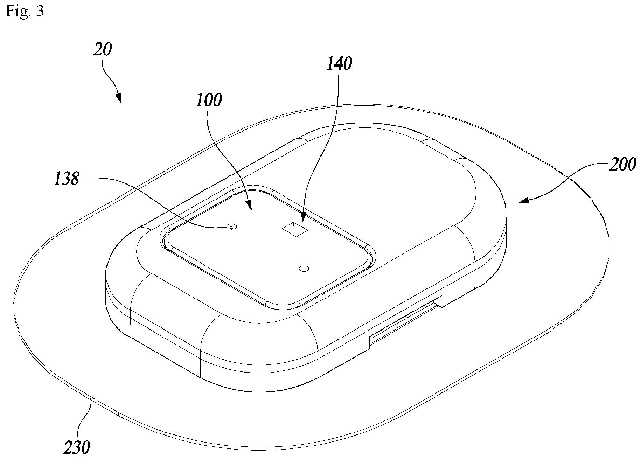

FIG. 1 is a perspective view showing an applicator assembly according to an embodiment of the present disclosure, FIG. 2 is a perspective view showing the body attachable unit attached to the skin of a user, and FIG. 3 is a perspective view showing the body attachable unit.

An applicator assembly (10) according to an embodiment of the present disclosure includes a sensor unit (100) including a sensor (110) that is inserted into the skin of a user and measures biometric information, and a base unit (200) constituting body attachable unit (20) with sensor unit (100), and an applicator (30) for attaching the body attachable unit (20) to the skin of a user. The applicator assembly (10) may be provided to a user with the sensor unit (100) and the base unit (200) mounted separately on the applicator (30). The sensor unit (100) and the base unit (200) can be automatically assembled and attached to the skin of a user by the operation of the applicator (30).

As shown in FIG. 2, the body attachable unit (20) can measure biometric information by being attached to the skin of a user, and wirelessly transmit the measured data to an external terminal (5). The external terminal (5) may be a variety of devices capable of receiving measurement data from the body attachable unit (20), such as a portable terminal, a medical device, a PC, or a server. Biometric information that the body attachable unit (20) can measure is not limited to specific information. As an exemplary embodiment, the body attachable unit (20) may periodically measure blood glucose level for a user and transmit the blood glucose measurement information to the external terminal (5).

As shown in FIG. 3, the body attachable unit (20) includes the sensor unit (100) for measuring the biometric information of a user, and the base unit (200) that is attached to the skin of a user and coupled to the sensor unit (100). The base unit (200) may have electronic components installed therein. This base unit (200) is electrically connected to the sensor unit (100) and can process biometric information measured by the sensor unit (100) and transmit it to the external terminal (5).

As another embodiment, the base unit (200) may be configured to simply support the sensor unit (100) so that it is not separated from the skin of a user. In this case, a separate electronic unit that can process biometric information measured by the sensor unit (100) and transmit it to the external terminal (5) may be detachably coupled to the base unit (200).

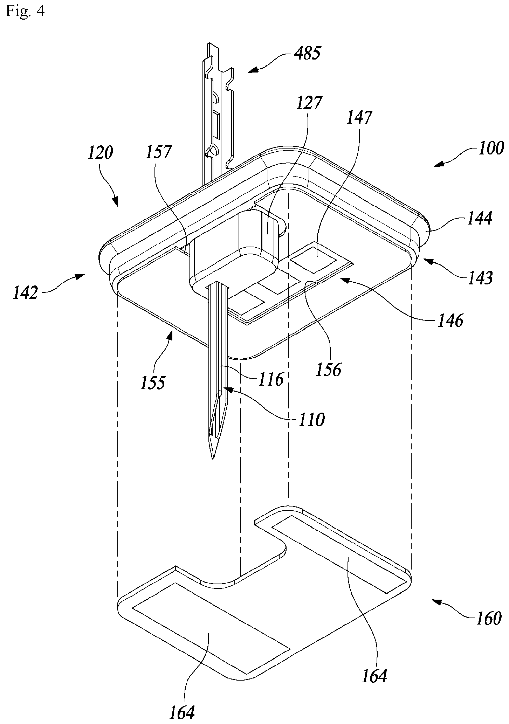

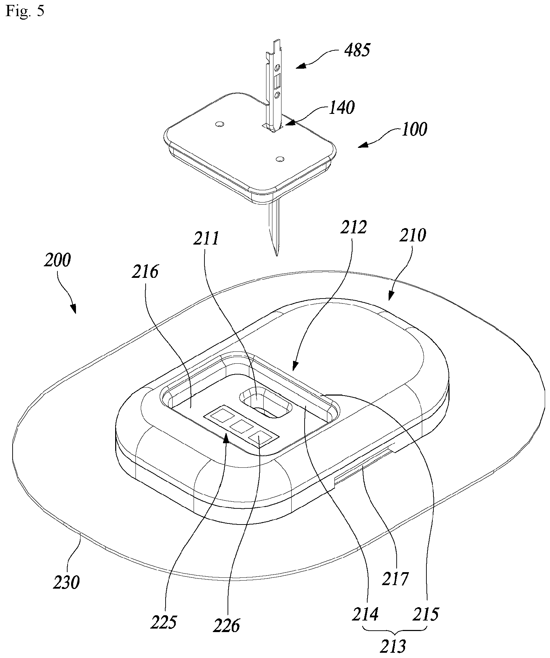

As shown in FIGS. 3 to 5, the sensor unit (100) includes the sensor (110) inserted into the skin of a user, a sensor unit housing (120) to which the sensor (110) is coupled, a sensor unit-electrical contact portion(146) electrically connected to the sensor (110) coupling to the sensor unit housing(120), an adhesive layer (155) provided on the sensor unit housing (120) so as to be attached to the base unit (200), a protective sheet (160) covering the adhesive layer (155).

A portion of the sensor (110) is disposed inside the sensor housing (120) and is electrically connected to the sensor unit-electrical contact portion (146). The sensor (110) has an insertion portion (116) that protrudes from the sensor housing (120) and can be inserted into the skin of a user.

In the sensor unit housing (120), a housing opening (140) through which a part of the sensor (110) and a needle (485) for inserting the sensor (110) into the skin of a user is inserted is formed to penetrate the sensor unit housing (120) in the thickness direction. The sensor unit housing (120) includes a housing body (142) and a boss (127) protruding from one side of the housing body (142). The housing opening (140) is formed to penetrate the housing body (142) and the boss (127). The housing body (142) is shaped to fit into the base unit recess (213) of the base unit (200). The housing body (142) includes a body portion (143) provided with a boss (127) and a cover portion (144) that is wider than the body portion (143).

The sensor unit housing (120) is not limited to the configuration shown, and may be changed to various other configurations on which the sensor (110) may be mounted and coupled to the base unit (200).

The sensor unit-electrical contact portion (146) is placed in the sensor unit housing (120) to be electrically connected to the sensor (110). The sensor unit-electrical contact portion (146) may include a plurality of terminal portions (147) for transmitting electrical signals. Some part of the sensor unit-electrical contact portion (146) is exposed to one surface of the sensor unit housing (120) so that the sensor (110) and a base unit- electrical contact portion (225) of the base unit (200) can be electrically connected.

An adhesive layer (155) is placed on the surface of the sensor unit housing (120). The adhesive layer (155) has adhesive properties on both sides. One side of the adhesive layer (155) is adhered to the sensor unit housing (120), and the other side of the adhesive layer (155) is covered with the protective sheet (160). The adhesive layer (155) may be attached to the base unit (200) after the protective sheet (160) is separated. In the middle portion of the adhesive layer (155), an adhesive layer hole (156) and an adhesive layer opening (157) are formed to penetrate the adhesive layer (155) in the thickness direction.

The protective sheet (160) covers and protects the adhesive layer (155). The protective sheet (160) is made of a material that is detachably attached to the adhesive layer (155). If the adhesive layer (155) is left exposed to the air for a long time, the adhesiveness of the adhesive layer (155) may deteriorate. The protective sheet (160) covers the adhesive layer (155) to prevent the problem of reducing of adhesiveness of the adhesive layer (155), and it facilitates handling of the sensor unit (100) by an operator during the manufacturing process of the sensor unit (100) or the process of assembling the sensor unit (100) to the applicator (30).

An adhesive member (164) is provided on the surface of the protective sheet (160). The adhesive member (164) may be in the form of a double-sided tape with adhesive properties on both sides. One side of the adhesive member (164) may be adhered to the protective sheet (160), and the other side of the adhesive member (164) may be adhered to the removing member (610) provided in the applicator (30). Accordingly, the protective sheet (160) can be coupled to the removing member (610) through the adhesive member (164).

The sensor unit (100) is mounted on the applicator (30) with the protective sheet (160) attached to the adhesive layer (155). The protective sheet (160) may be separated from the adhesive layer (155) by a removing unit (600) of the applicator (30) before the sensor (110) is inserted into the skin of a user. The sensor unit (100) moves toward the base unit (200) with the protective sheet (160) separated and is coupled to the base unit (200).

The base unit (200) includes a base unit housing (210) to which the sensor unit (100) is coupled, and electronic components installed inside the base unit housing (210). The electronic component may include a base unit electrical contact portion (225) in contact with the sensor unit-electrical contact portion (146) of the sensor unit (100), a circuit board (223), a battery (228), a processor chip for processing signals, and a communication chip for wireless communication with the external terminal (5), etc. The base unit-electrical contact portion (225) may include a plurality of terminal portions (226) for transmitting electrical signals.

The base unit housing (210) is provided with an insertion hole (211) through which the insertion portion (116) of the sensor (110) and the needle (485) can pass, and a mounting portion (212) in which the sensor unit housing (120) is coupled. The mounting portion (212) includes a base unit recess (213) and a contact surface (216) provided inside the base unit recess (213). The base unit recess (213) may include a first recess (214) connected to the insertion hole (211), and a second recess (215) that is located farther from the insertion hole (211) than the first recess (214) and is connected to the first recess (214). The housing body (142) of the sensor unit (100) may be stably fitted and coupled to the base unit recess (213). A housing groove (217) is formed on the outer edge of the base unit housing (210). A portion of the locking hook (360) provided on the applicator (30) may be inserted into the housing groove (217).

An adhesive portion (230) is provided on the surface of the base unit housing (210). The adhesive portion (230) is attached to the surface of the lower housing (219) to adhere the base unit housing 210 to the skin of a user. The adhesive portion (230) may be covered and protected with a protective sheet. The protective sheet covering the adhesive portion (230) may be removed during the process of attaching the base unit (200) to the skin of a user.

The sensor unit (100) and the base unit (200) are installed in the applicator (30) in a separated state, and are coupled to each other in the process of operating the applicator (30) to insert the sensor (110) into the skin of a user, forming the body attachable unit (20). The sensor unit (100) may be mounted on the applicator (30) while the needle (485) coupled, and moved toward the base unit (200) with the needle (485) coupled thereto to be coupled to the base unit (200). After the sensor unit (100) is coupled to the base unit (200), the needle (485) is separated from the sensor unit (100), and only the body attachable unit (20) remains on the skin of a user.

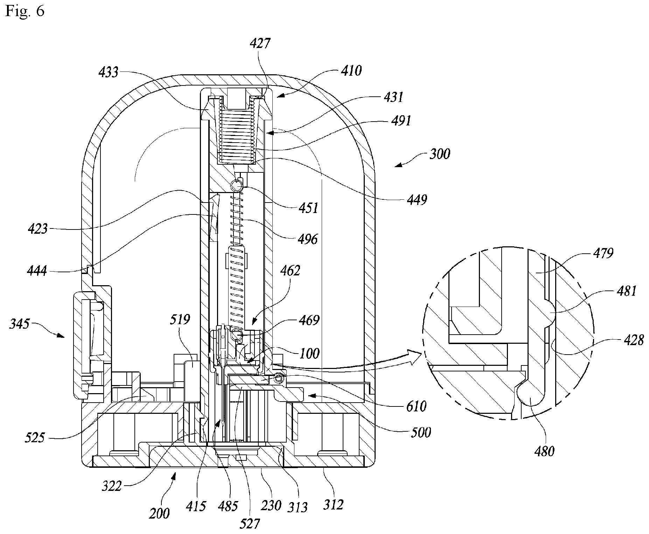

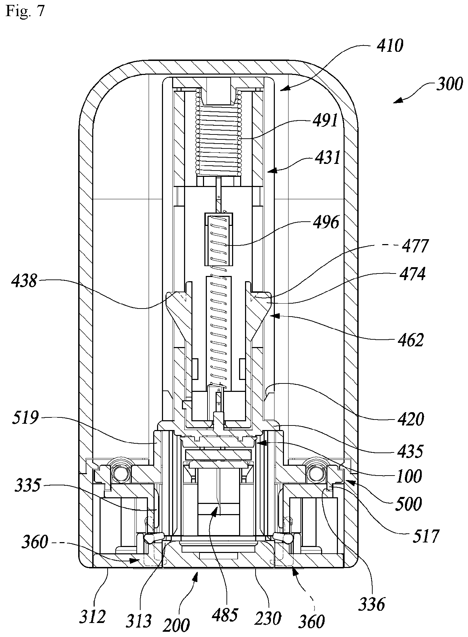

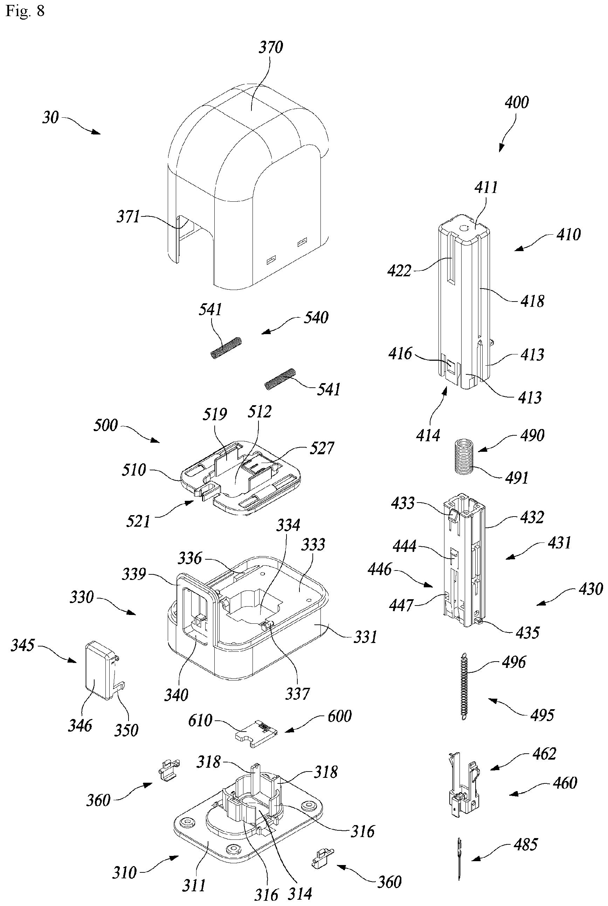

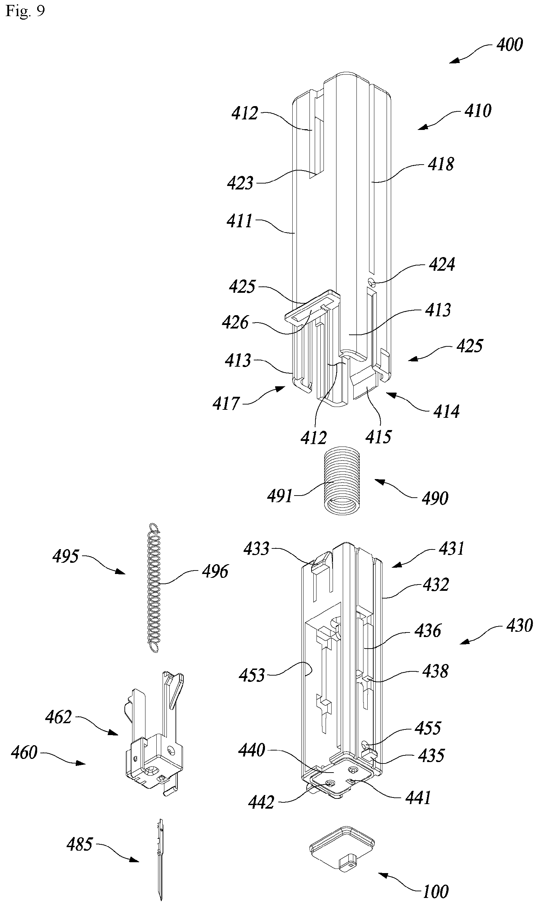

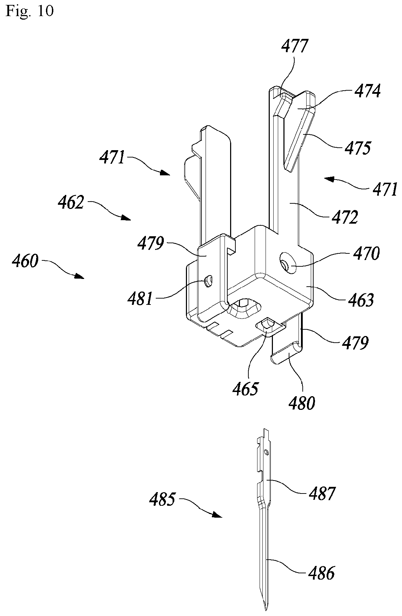

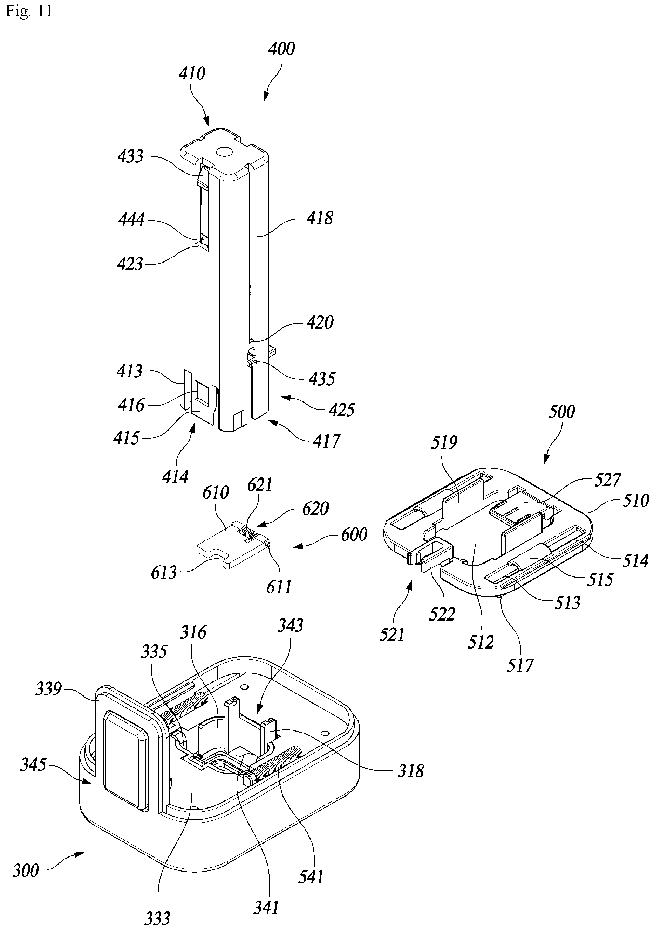

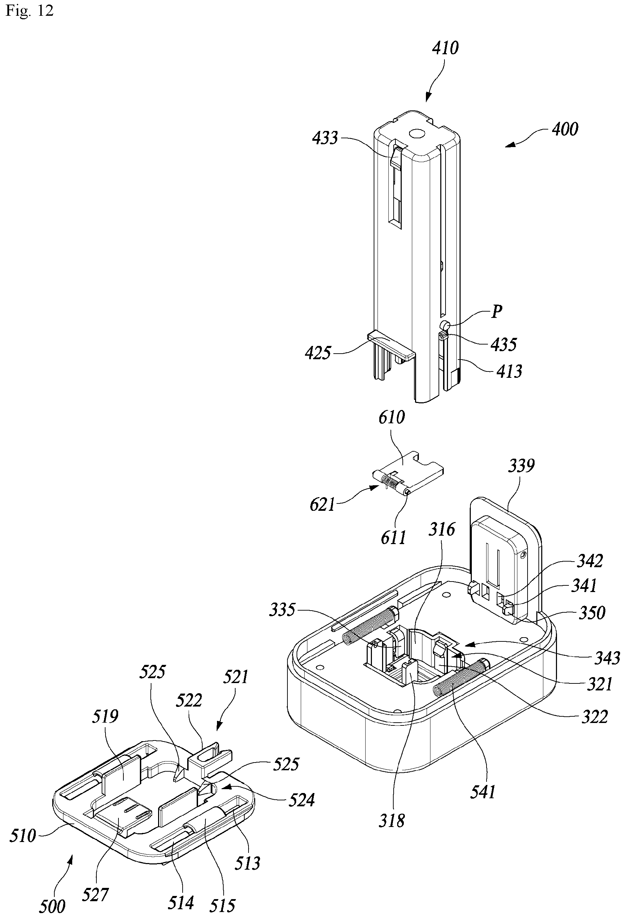

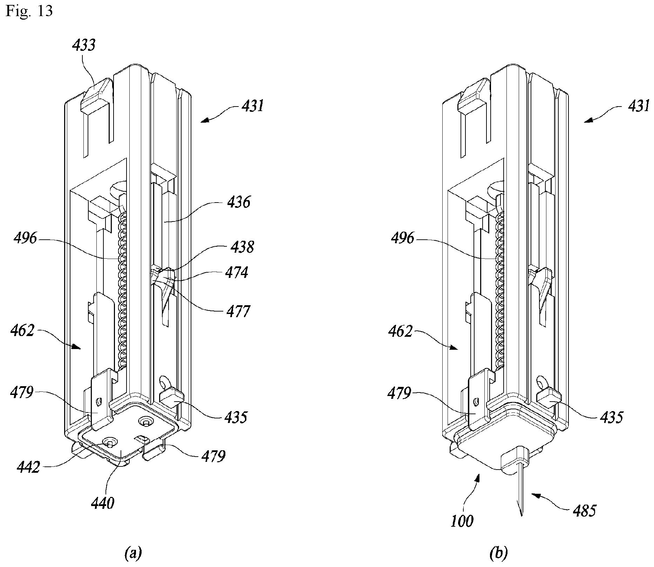

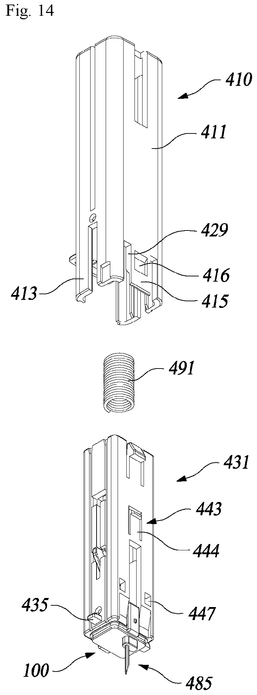

Referring to FIGS. 6 to 20, the applicator (30) operates with the sensor unit (100) and the base unit (200) mounted, thereby coupling the sensor unit (100) and the base unit (200) and can attach the body attachable unit (20) in which the sensor unit (100) and the base unit (200) are coupled to the skin of a user. The applicator (30) is separated from the body attachable unit (20) after attaching the body attachable unit (20) to the skin of a user, and the body attachable unit (20) may remain attached to the skin of a user by the adhesive portion (230).

The applicator (30) includes an applicator body (300) to which the base unit (200) is detachably coupled, an insertion unit assembly (400) that includes an insertion unit (430) for inserting the sensor (110) into the skin of a user, a stopper member (500) movably installed on the applicator body (300), and a removing unit (600) for separating the protective sheet (160) of the sensor unit 100. The insertion unit (430) mounted on the column member (410) coupled to the applicator body 300 and may move the sensor unit (100) from a first position spaced apart from the base unit (200) by a preset distance to a second position coupled with the base unit (200). The sensor unit (100) and the base unit (200) are coupled at the second position to form the body attachable unit (20).

The applicator body (300) includes a base frame (310), a middle frame (330) disposed on the base frame (310), and a top case (370) that is coupled to the middle frame (330) and covers the upper part of the middle frame (330).

The base frame (310) includes a frame base portion (311) having a bottom portion (312) that can be in contact with the skin of a user. The bottom portion (312) of the frame base portion (311) is provided with a recess (313) into which the base unit (200) is mounted. The base unit (200) is detachably coupled to the recess (313) so that the adhesive portion (230) can face the skin of a user and placed in a second position spaced apart from the sensor unit (100). A base frame opening (314) is formed in the middle of the frame base portion (311) to penetrate the frame base portion (311) in the thickness direction. A fence portion (316) and a support (318) are provided to protrude around the base frame opening (314). When the base frame (310) is coupled to the middle frame (330), the fence portion (316) and the support (318) are inserted into the middle frame opening (334) of the middle frame (330). The fence portion (316) may support the column member (410) in contact with the outer surface of the column member (410) provided in the insertion unit assembly (400). The fence portion (316) may be provided in an elastically deformable form so as to be in stable close contact with the outer surface of the column member (410). The support (318) supports the removing member (610) of the removing unit (600). An applicator body snap portion (321) is provided inside the fence portion (316) to secure the column member (410). The applicator body snap portion (321) includes a snap hook (322) engaged with the column member (410).

Locking hooks (360) for detachably fixing the base unit (200) are coupled to both sides of the base frame opening (314) of the frame base portion (311). The locking hook (360) may engage the base unit (200) which is mounted in the recess (313) and secure the base unit (200) without being separated from the recess (313). The locking hook (360) may be disengaged from the base unit (200) after the body attachable unit (20) is attached to the skin of a user. The locking hook (360) is pivotally coupled to the base frame (310) and can be tilted within a certain angle range. The locking hook (360) includes a pivot portion (361) rotatably coupled to the base frame (310), a hook portion (362) connected to the pivot portion (361), and a lever portion (365) protruding from the pivot portion (361). The hook portion (362) may be inserted into the housing groove (217) of the base unit (200) and engaged with the base unit (200). The lever portion (365) protrudes from the pivot portion (361) toward the base frame opening (314). The locking hook (360) may be disengaged from the base unit (200) by rotating around the pivot portion (361) and being tilted when the lever portion (365) is pressed by the shuttle (431) of the insertion unit (430).

The locking hook (360) is not limited to the shown configuration and can be changed to various other configurations.

In addition, the specific configuration of the base frame (310) is not limited to be shown and can be changed in various ways.

The middle frame (330) includes an outer frame (331) coupled to the base frame (310), a stage (333) disposed inside the outer frame (331), and a support fixture (339) that protrudes higher than the stage (333). The stage (333) is positioned on the frame base portion (311). In the middle of the stage (333), a middle frame opening (334) into which the fence portion (316) and the support (318) of the base frame (310) are inserted is provided. The middle frame opening (334) is formed to correspond to the base frame opening (314) of the base frame (310). That is, when the base frame (310) and the middle frame (330) are coupled, the middle frame opening (334) and the base frame opening (314) are connected. The middle frame opening (334) is connected to the base frame opening (314) and forms an applicator body groove (341).

The applicator body groove (341) constitutes an applicator body coupling portion (343) for coupling the column member (410) of the insertion unit assembly (400) to the applicator body (300) along with the fence portion (316) and the applicator body snap portion (321). A portion of the column member (410) is inserted into the applicator body groove (341) so that the insertion unit assembly (400) can be assembled to the applicator body (300).

Elastic arms (335) which support the locking hook (360) are provided on both sides of the middle frame opening (334). The elastic arm (335) is installed to be in contact with the locking hook (360) and can apply an elastic force to the locking hook (360) in a direction in which it engages the base unit (200). That is, the elastic arm (335) can bias the locking hook (360) in a direction of engaging with the base unit (200) by applying elastic force in contact with one side of the locking hook (360).

The stage (333) is provided with a guide rail (336) and a fixing protrusion (337). The guide rail (336) plays a role in guiding the stopper member (500) linearly. The fixing protrusion (337) supports the stopper member driver (540), which provides mobility to the stopper member (500).

The support fixture (339) supports the operating member (345) for operating the stopper member (500). The support fixture (339) is provided with a support fixture groove (340) for the installation of the operating member (345) and a plurality of support fixture openings (341 and 342).

The operating member (345) includes a button portion (346), a pressing protrusion (348) protruding from the button portion (346), and an operating member hook (350). The operating member (345) is operated by a user to operate the stopper member (500). The operating member (345) may move in the support fixture groove (340) without separating from the support fixture (339) by inserting the operating member hook (350) into the support fixture opening (341) of the support fixture (339). When the operating member (345) is pressed toward the inside of the applicator body (300) by a user, the pressing protrusion (348) may press the fixing hook (522) of the stopper member (500). The stopper member (500) may be fixed to the applicator body (300) by a fixing hook (522), and then, when the operating member (345) presses the fixing hook (522), the fixing force on the applicator body (300) is released and it can move.

In addition to the illustrated configuration, the operating member (345) may be changed to various other configurations installed on the applicator body (300) to be operated by a user to move the stopper member (500).

The top case (370) is coupled with the middle frame (330) and covers the upper part of the middle frame (330). On one side of the top case (370), a top case opening (371) is provided in a shape which allows the support fixture (339) of the middle frame (330) to be coupled.

The specific configuration of the top case (370) is not limited to that shown and can be changed in various ways. Additionally, the method of coupling the top case (370) and the middle frame (330) can also be changed in various ways.

In addition to the configuration including the base frame (310), the middle frame (330), and the top case (370) as shown, the applicator body (300) may be changed to various other configurations that can support the insertion unit assembly (400) by being coupled with the insertion unit assembly (400). Additionally, the applicator body (300) may be coupled to the base unit (200) in various other ways other than using a locking hook.

The insertion unit assembly (400) includes a column member (410) fixed to the applicator body (300) and an insertion unit (430) that is supported on the column member (410) to be coupled with the sensor unit (100) and moves the sensor unit (100) from the first position to the second position.

The column member (410) is fixed to the applicator body (300) and supports the insertion unit (430). The column member (410) includes a column member body (411). The column member body (411) has a space inside to accommodate the insertion unit (430), and has a column member opening (412) opened to the outside at one end. One end of the column member body (411) is provided with a coupling end portion (417) that is inserted into the applicator body groove (341) of the applicator body (300). The coupling end portion (417) includes a plurality of column member legs (413). A plurality of column member legs (413) are provided to surround the column member opening (412). The column member leg (413) may be provided in an elastically deformable form. The column member (410) may be coupled to the applicator body (300) in such a way that the coupling end portion (417) is inserted into the applicator body groove (341) of the applicator body (300). In the process of inserting the coupling end portion (417) into the applicator body groove (341), the plurality of column member legs (413) are elastically deformed, so that the coupling end portion (417) can be more smoothly inserted into the applicator body groove (341).

One end of the column member body (411) is provided with a column member snap portion (414) that can engage with the applicator body snap portion (321) of the applicator body (300). The column member snap portion (414) constitutes a column member coupling portion (425) coupled to the applicator body coupling portion (343) together with the coupling end portion (417). The column member (410) can be more firmly fixed to the applicator body (300) by engaging the column member snap portion (414) with the applicator body snap portion (321). The column member snap portion (414) includes an elastically deformable snap arm (415). The snap arm (415) is provided with a snap groove (416) into which the snap hook (322) of the applicator body snap portion (321) engages. When the column member (410) is inserted into the applicator body groove (341), the snap hook (322) and the snap arm (415) come into contact with each other, and a portion of the snap hook (322) is inserted into the snap groove (416).

The column member (410) can be fixed stably to the applicator body (300) by engaging with the snap hook (322) and the snap arm (415) in a state in which the coupling end portion (417) is inserted into the applicator body groove (341) of the applicator body (300). Additionally, the fence portion (316) of the applicator body (300) is in close contact with the outer surface of the column member (410), so that the column member (410) can be supported more stably. In this way, the column member (410) can be simply coupled to the applicator body (300) by inserting one end into the applicator body groove (341) of the applicator body (300).

The method of coupling the applicator body (300) and the column member (410) is not limited to that shown and may be changed in various ways.

Slits (418) are formed on both sides of the column member body (411) in a direction parallel to the moving direction of the sensor unit (100). A release portion (420) is provided in the middle portion of the slit (418). The release portion (420) is a part where a portion of the carrier (462) may contact while the carrier (462) of the insertion unit (430) moves toward the base unit (200). The carrier (462) may be disengaged from the shuttle (431) of the insertion unit (430) by the action of the release portion (420).

Additionally, guide grooves (422) are formed on the other both sides of the column member body (411) in a direction parallel to the moving direction of the sensor unit (100). The shuttle hook (433) of the shuttle (431) is placed in the guide groove (422), so that the shuttle hook (433) can move along the guide groove (422). A column member detent (423) is provided at the end of the guide groove (422) to limit the moving distance of the shuttle (431). When the shuttle (431) moves from the first position with the sensor unit (100) and reaches the second position, the shuttle hook (433) comes into contact with the column member detent (423) so that the shuttle (431) may stop at the second position. A column member hole (424) is formed on one side of the column member body (411) to penetrate through the side of the column member body (411) from inside to outside. A safety pin (P) may be inserted into the column member hole (424) to restrict the movement of the shuttle (431) and the carrier (462). A cover portion (425) is provided on another side of the column member body (411). The cover portion (425) is provided with a cover groove (426) into which the removing member (610) can be partially inserted. A retention portion (427) is provided inside the column member (410). The retention portion (427) serves to support the shuttle driver (490) that provides moving force to the shuttle (431).

The insertion unit (430) is coupled to the applicator body (300) through the column member (410) and can move the sensor unit (100) from the first position to the second position. The insertion unit (430) includes a shuttle (431) movably installed inside the column member (410) and a needle assembly (460) having a needle (485) that can be moved with the shuttle (431) and inserted into the skin of a user.

The shuttle (431) is installed to be linearly movable inside the column member (410). In a state in which the column member (410) is coupled to the applicator body (300), the shuttle (431) can move from the first position to the second position with the sensor unit (100). The shuttle (431) includes a shuttle body (432) with a space inside to accommodate the needle assembly (460). A shuttle hook (433) is provided on one side of the shuttle body (432) to limit the movement range of the shuttle (431). The shuttle hook (433) may restrict the movement of the shuttle (431) by contacting the column member detent (423) of the column member (410) when the shuttle (431) moves to the second position. A shuttle protrusion (435) is provided on the other side of the shuttle (431). The shuttle protrusion (435) protrudes from the shuttle body (432) so as to contact the stopper member (500). The shuttle (431) is fixed in the first position when the shuttle protrusion (435) comes into contact with the stopper member (500), and can move to the second position when the shuttle protrusion (435) leaves the stopper member (500). The shuttle (431) can move the locking hook (360) by the shuttle protrusion (435). That is, when the shuttle (431) reaches the second position, the shuttle protrusion (435) presses the lever portion (365) of the locking hook (360), so that the locking hook (360) may rotates and be disengaged from the base unit (200).

A pair of slits (436) are formed on both sides of the shuttle body (432) in a direction parallel to the moving direction of the shuttle (431). The middle portion of the slit (436) is provided with a shuttle detent (438) with which the carrier latch (477) of the carrier (462) can be engaged. In a state in which the carrier latch (477) engaged with the shuttle detent (438), the carrier (462) moves with the shuttle (431) and cannot relatively move with respect to the shuttle (431). And when the carrier latch (477) is disengaged from the shuttle detent (438), the carrier (462) can relatively move with respect to the shuttle (431).

A shuttle bottom portion (440) in contact with the sensor unit (100) is provided at one end of the shuttle body (432). The shuttle bottom portion (440) is provided with a shuttle through hole (441) and a pressing protrusion (442). A needle (485) which is movable inside the shuttle body (432) may protrude to the outside of the shuttle body (432) through the shuttle through hole (441). The pressing protrusion (442) is provided to be inserted into the sensor unit groove (138) of the sensor unit (100). When the sensor unit (100) is mounted on the insertion unit (430), the pressing protrusion (442) is inserted into the sensor unit groove (138) of the sensor unit (100), so that the sensor unit (100) can maintain its fixed position without moving.

The shuttle body (432) is provided with a clamping portion (443) for temporarily fixing the shuttle (431) to the column member (410). The clamping portion (443) includes a clamping hook (444) elastically deformably connected to one side of the shuttle body (432). The clamping hook (444) may be used to temporarily fix the shuttle (431) to the column member (410) during the process of assembling the insertion unit assembly (400). The insertion unit assembly (400) is coupled to the applicator body (300) in a state in which the shuttle (431) is temporarily fixed to the column member (410). In a state in which the shuttle (431) is temporarily fixed to the column member (410) by the clamping hook (444), the shuttle (431) cannot move. When the insertion unit assembly (400) is coupled to the applicator body (300) with the shuttle (431) temporarily fixed to the column member (410), the insertion unit (430) does not operate even if a user presses the operating member (345). Therefore, in order for the applicator (30) to be in an operable state, it is necessary that the insertion unit assembly (400) is coupled to the applicator body (300) and the temporarily fixed state of the shuttle (431) is released. In the process of assembling the insertion unit assembly (400), when the shuttle (431) is inserted into the interior of the column member (410) and the he clamping hook (444) is engaged with the column member detent (423) of the column member (410), the shuttle (431) is temporarily fixed to the column member (410). At this time, since the clamping hook (444) is engaged with the column member detent (423), the shuttle (431) cannot move.

There are various advantages to temporarily fixing the shuttle (431) to the column member (410) during the process of assembling the insertion unit assembly (400). That is, if the shuttle (431) is temporarily fixed to the column member (410), it is possible to prevent the shuttle (431) from accidentally moving during the process of assembling the insertion unit assembly (400) or coupling the insertion unit assembly (400) to the applicator body (300). Additionally, since a sharp needle (485) is coupled to the shuttle (431), the risk of a safety accident caused by accidental movement of the needle (485) during the manufacturing process can be reduced.

In the drawing, the shuttle (431) is shown to be temporarily fixed to the column member (410) in such a way that the clamping hook (444) provided on the shuttle (431) is engaged with the column member detent (423) of the column member (410), but the method for temporary fixing the shuttle (431) may be changed in various ways. As another exemplary embodiment, the clamping portion may be provided on the column member (410) to engage with the shuttle (431). Additionally, it is possible to temporarily fix the shuttle (431) to the column member (410) using various other types of components in addition to the elastically deformable clamping hook.

A shuttle locking portion (446) is provided on one side of the shuttle body (432). The shuttle locking portion (446) is intended to fix the shuttle (431) reaching the second position in the second position.

After the applicator (30) operates and inserts the sensor (110) into the skin of a user, the applicator (30) needs to be separated from the body attachable unit (20) and disposed of. Since the needle (485) of the applicator (30) is inserted into the skin of a user in the process of inserting the sensor 110 into the skin of a user and then comes out of the skin of a user, the needle (485) is polluted after using the applicator (30). Additionally, after use of the applicator (30), in the case in which the shuttle (431) is moved to the first position and reloaded, the needle (485) may accidentally move, leading to a safety accident. To prevent this problem, the shuttle (431) is moved to the second position and then fixed in the second position by the shuttle locking portion (446). After use of the applicator (30), the applicator (30) cannot be reused because the shuttle (431) is fixed in the second position. The shuttle locking portion (446) includes a locking groove (447) into which the locking protrusion (525) of the stopper member (500) can be inserted. When the shuttle (431) reaches the second position inside the column member (410), the locking groove (447) faces the opening (429) of the column member (410).

A retention portion (449) and a fixing portion (451) are provided inside the shuttle (431). The retention portion (449) supports the shuttle driver (490). A needle release driver (495) that provides a moving force to the carrier (462) is coupled to the fixing portion (451).

In addition, the shuttle (431) includes a shuttle opening (453) and a shuttle hole (455). A safety pin (P) may be inserted into the shuttle hole (455). When the safety pin (P) passes through the column member hole (424) and is inserted into the shuttle hole (455), the shuttle (431) may be temporarily fixed to the column member (410). The shuttle (431) can be temporarily fixed to the column member (410) by inserting the safety pin (P) into the shuttle hole (455).

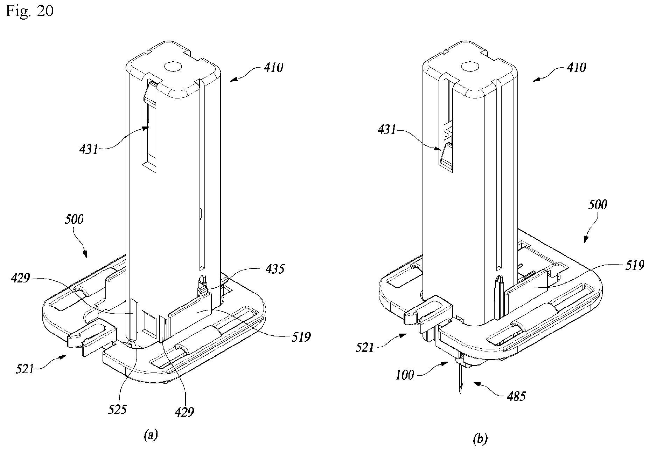

The shuttle (431) moves by receiving movement force from the shuttle driver (490). The shuttle driver (490) includes an elastic member (491) that applies elastic force to the shuttle (431) in a direction in which it moves from the first position to the second position. The elastic member (491) has one end in contact with the retention portion (427) of the column member (410) and the other end in contact with the retention portion (449) of the shuttle (431). The shuttle (431) may maintain a state in which the elastic member (491) is elastically deformed and temporarily fixed to the column member (410) by the stopper arm (444) and the safety pin (P). After the insertion unit assembly (400) is coupled to the applicator body (300), the stopper arm (444) is disengaged from the column member detent (423) of the column member (410), and when the safety pin (P) is separated from the shuttle (431), the shuttle protrusion (435) comes into contact with the stopper member (500), and the shuttle (431) is converted to a movable state by the operating member (345). As shown in (a) of FIG. 20, when the stopper member (500) comes into contact with the shuttle protrusion (435) and supports the shuttle protrusion (435), the shuttle (431) may be fixed in the first position. And, as shown in (c) of FIG. 20, when the stopper member (500) moves and deviates from the shuttle protrusion (435), the shuttle (431) moves to the second position by the elastic force of the elastic member (491).

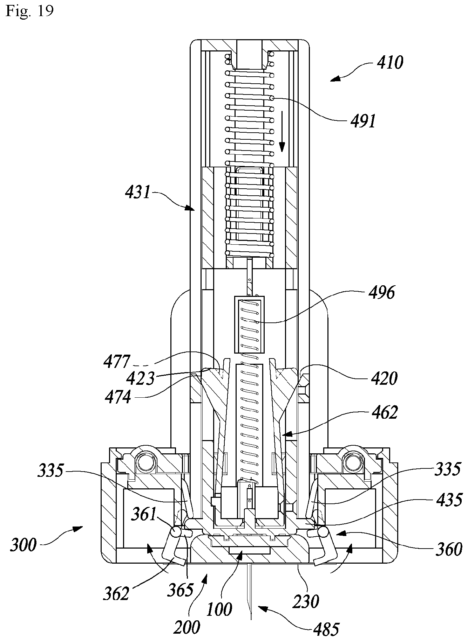

The shuttle (431) may move the sensor unit (100) toward the base unit (200) by moving from the first position to the second position, and release the locking hook (360) when it reaches the second position. As shown in FIG. 19, when the shuttle (431) reaches the second position, the shuttle protrusion (435) presses the lever portion (365) of the locking hook (360). At this time, the locking hook (360) is tilted to disengage from the base unit housing (210) while elastically deforming the elastic arm (335) of the applicator body (300). Accordingly, the base unit (200) may be disengaged from the locking hook (360) and separated from the applicator body (300).

The shuttle driver (490) may be changed to various other configurations that can provide a moving force to the shuttle (431) in addition to the configuration including the elastic member (491) in the form of a coil spring.

The needle assembly (460) includes a carrier (462) to which the sensor unit (100) is detachably coupled, and a needle (485) coupled to the carrier (462) to penetrate the sensor unit (100). The needle (485) may be coupled to the sensor unit (100) and move forward from the first position to the second position with the sensor unit (100) to be inserted into the skin of a user, and may be pulled back together with the carrier (462) at the second position to be separated from the skin of a user and the sensor unit (100).

The carrier (462) is coupled to the shuttle (431) to be relatively movable. A portion of the carrier (462) engages with the shuttle (431) so that it can advance together with the shuttle (431) from the first position to the second position. Then, the carrier (462) is disengaged from the shuttle (431) in the second position and may retreat in a direction away from the sensor unit (100).

The carrier (462) includes a carrier body (463) to which the needle (485) is coupled, an elastically deformable carrier wing (471) extending from the carrier body (463), and a grip arm (479) connected to the side of the carrier body (463).

An insertion groove (465) is formed on one side of the carrier body (463). A portion of the needle (485) may be inserted into the insertion groove (465). A clamp portion may be provided in the insertion groove (465) to engage with the needle (485) and secure the needle (485). A fixing portion (469) to which the needle release driver (495) is coupled is provided on one side of the carrier body (463). Carrier (462) can relatively move with respect to shuttle (431) by needle release driver (495).

The needle release driver (495) provides a moving force to the carrier (462). The needle release driver (495) includes a needle release elastic member (496) whose one end is coupled to the fixing portion (451) of the shuttle (431) and the other end is coupled to the fixing portion (469) of the carrier (462). The needle release elastic member (496) is in the form of a tension spring and applies an elastic force to pull the carrier (462) toward the fixing portion (451) of the shuttle (431). That is, the needle release elastic member (496) can apply an elastic force to the carrier (462) in a direction away from the sensor unit (100) to move the needle (485) coupled to the carrier (462) to be separated from the sensor unit (100).

The needle release driver (495) can be changed to various other configurations that can provide a moving force to the carrier (462) in addition to the configuration including the needle release elastic member (496) in the form of a coil spring.

A carrier hole (470) is formed on the side of the carrier body (463) to penetrate the side of the carrier body (463) from inside to out. A safety pin (P) may be inserted into the carrier hole (470). The safety pin (P) sequentially passes through the column member hole (424) of the column member (410) and the shuttle hole (455) of the shuttle (431) and then is inserted into the carrier hole (470) thereby temporarily fixing the carrier (462) to the shuttle (431).

In the process of assembling the insertion unit assembly (400) by temporarily fixing the shuttle (431) and the carrier (462) using a safety pin (P), or in the process of coupling the insertion unit assembly (400) to the applicator body (300), a problem in which the shuttle (431) and the carrier (462) are accidentally moved may be reduced.

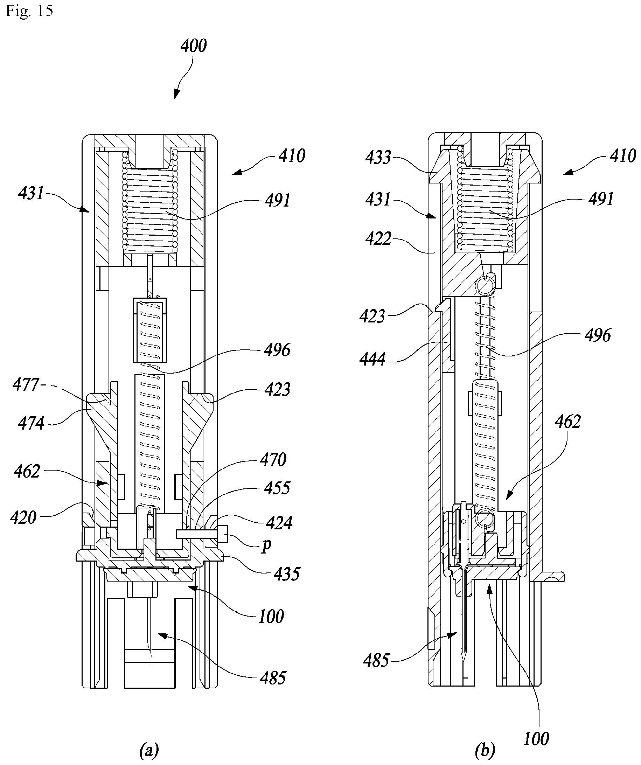

As shown in (a) of FIG. 15, the safety pin (P) can be inserted to the column member hole (424) of the column member (410), the shuttle hole (455) of the shuttle (431), and the carrier hole (470) of the carrier (462). In a state in which the column member hole (424), the shuttle hole (455), and the carrier hole (470) are aligned in a line, the safety pin (P) sequentially may pass through the column member hole (424) and the shuttle hole (455), and then be inserted to the carrier hole (470). At this time, the shuttle (431) and the carrier (462) cannot move. Therefore, the safety pin (P) can prevent accidental movement of the shuttle (431) and the carrier (462) during the process of assembling the insertion unit assembly (400) or coupling the insertion unit assembly (400) to the applicator body (300). When the safety pin (P) is separated from the carrier (462), the shuttle (431), and the column member (410), the insertion unit (430) becomes operable by the operating member (345).

The specific configuration and number of safety pins (P) can be changed in various ways. As another exemplary embodiment, a safety pin passing through the column member may be installed to engage the shuttle without reaching the carrier. In this case, the safety pin restricts the movement of the shuttle in the first position to prevent malfunction in the applicator caused by user carelessness.

Additionally, the use of safety pins (P) may be excluded in the manufacturing process of the applicator (30). In this case, the shuttle (431) can be temporarily fixed to the column member (410) using the clamping hook (444).

The carrier wing (471) is connected to the carrier body (463) in a shape that extends from the carrier body (463) in the moving direction of the carrier (462). The carrier wing (471) includes a wing body (472) elastically deformably connected to the carrier body (463), and a trigger (474) and a latch portion (477) protruding from the wing body (472).

The trigger (474) is provided on one side of the wing body (472) to protrude outward from the wing body (472). The trigger (474) is inserted into the slit (436) of the shuttle (431) and the slit (418) of the column portion (410) and can move along these slits (436 and 418). The carrier (462) comes into contact with the release portion (420) of the column member (410) while moving from the first position to the second position together with the shuttle (431).

As shown in FIG. 19, the release portion (420) is placed in the middle of the slit (418) of the column member (410) to press the trigger (474). While the carrier (462) is moving from the first position to the second position, the trigger (474) may come into contact with the release portion (420) and the carrier wing (471) may be elastically deformed. The trigger (474) is provided with a trigger inclined portion (475) that contacts the release portion (420).

The carrier latch (477) protrudes outward from the wing body (472). Carrier latch (477) may engage shuttle detent (438) of shuttle (431). In a state in which the carrier latch (477) engaged with the shuttle detent (438), the carrier (462) can maintain the needle release elastic member (496) in an elastically deformed state and cannot move in the direction in which the elastic force of the needle release elastic member (496) acts. And when the trigger (474) contacts the release portion (420) and the carrier wing (471) is elastically deformed, the carrier latch (477) may be biased from the shuttle detent (438) and the carrier (462) may move.

The grip arm (479) is provided on the side of the carrier body (463) to be elastically deformable. A pair of grip arms (479) are arranged to face each other on both sides of the carrier body (463) and detachably fix the sensor unit housing (120) of the sensor unit (100). The grip arm (479) is provided with a grip protrusion (480) engaged with the sensor unit housing (120) and a protrusion (481) in contact with the inner wall (428) of the column member (410). The carrier (462) is coupled to the shuttle (431) so that the carrier body (463) is located inside the shuttle (431) and the end of the grip arm (479) protrudes from the shuttle (431). The carrier (462) may fix the sensor unit (100) in such a way that a pair of grip arms (479) engage with the sensor unit housing (120). At this time, the surface of the sensor unit housing (120) is in close contact with the shuttle bottom portion (440) of the shuttle (431), and the pressing protrusion (442) of the shuttle (431) is inserted into the sensor unit groove (138) of the sensor unit housing (120). Therefore, the sensor unit (100) can remain stably coupled to the shuttle (431).

And the carrier (462) may position the sensor unit (100) in the first position when the insertion unit assembly (400) is coupled to the applicator body (300), as shown in FIG. 6. When the carrier (462) is located inside the column member (410), the protrusion (481) of the grip arm (479) comes in contact with the inner wall (428) of the column member (410). Accordingly, the grip arm (479) is biased in a direction in which it engages the sensor unit housing (120), and the carrier (462) can remain stably coupled to the sensor unit (100).

Meanwhile, when the sensor unit (100) and the carrier (462) move toward the base unit (200) and the sensor unit (100) is coupled to the base unit (200), the protrusion (481) deviates from the inner wall (428) of the column member (410). Therefore, in a state in which the sensor unit (100) is coupled with the base unit (200), the fixing force of the grip arm (479) is weakened, and when the carrier (462) moves by the needle release driver (495), the grip arm (479) can be separated from the sensor unit (100).

In addition to the configuration shown, the carrier (462) can be changed to various other configurations in which it is coupled to the needle (485) and installed to be relatively movable with respect to the shuttle (431).

The needle (485) is fixed to the carrier (462) and can move from the first position to the second position while coupled to the sensor unit (100). When the sensor unit (100) moves to the second position, the needle (485) penetrates the skin of a user before the sensor (110) and allows the sensor (110) to be stably inserted into the skin. The needle (485) is separated from the skin of a user after the sensor (110) is inserted into the skin of a user. The needle (485) includes a needle body (486) that can be inserted into the skin of a user, and a needle head (487) coupled to the carrier (462).

The specific configuration of the needle (485) is not limited to that shown and may be changed in various ways. Additionally, the method in which the needle (485) is coupled to the carrier (462) may also vary.

The insertion unit assembly (400) may be coupled to the applicator body (300) in a state in which the elastic member (491) of the shuttle driver (490) which is for moving the shuttle (431) and the needle release elastic member (496) of the needle release driver (495) which is for moving the needle assembly (460) are elastically deformed. Additionally, the insertion unit assembly (400) may be coupled to the applicator body (300) while being coupled to the sensor unit (100). After the insertion unit assembly (400) is coupled to the applicator body (300), the safety pin (P) is removed and the stopper arm (444) is operated to release the restraining force on the shuttle (431) so that the insertion unit (430) can become operable by the operating member (345).

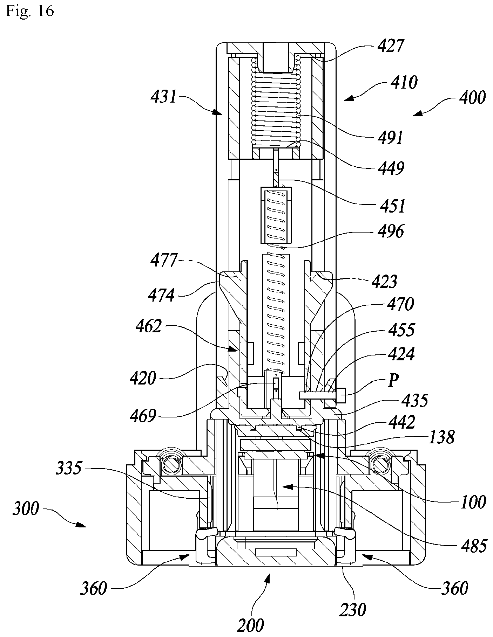

As shown in (a) and (b) of FIG. 15, The insertion unit assembly (400) may be temporarily fixed to the column member (410) so that the shuttle (431) maintains the position in which the elastic member (491) is elastically deformed by the clamping hook (444) and the safety pin (P), and may be assembled so that the carrier (462) is temporarily fixed to the shuttle (431) to maintain the position in which the needle release elastic member (496) is elastically deformed by the safety pin (P). In this way, the insertion unit assembly (400) in which the shuttle (431) and the carrier (462) are temporarily fixed is prevented from accidental movement of the shuttle (431) and the carrier (462), making it easy for an operator to handle during the assembly process. During the manufacturing process of the applicator assembly (10), an operator may assemble the insertion unit assembly (400) to which the shuttle (431) and the carrier (462) are temporarily fixed to the applicator body (300), as shown in FIG. 16.

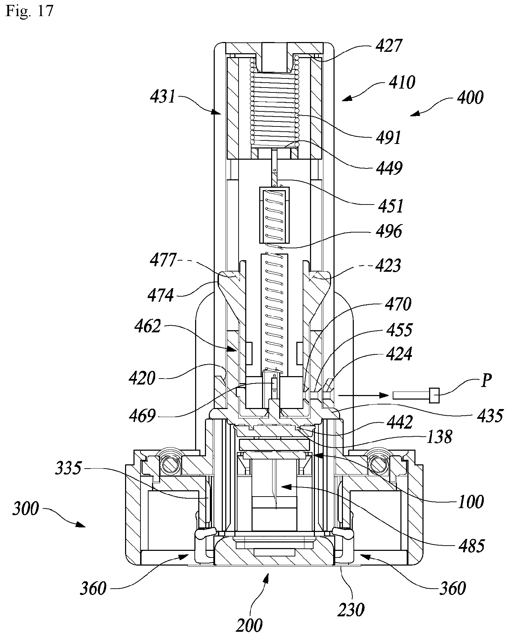

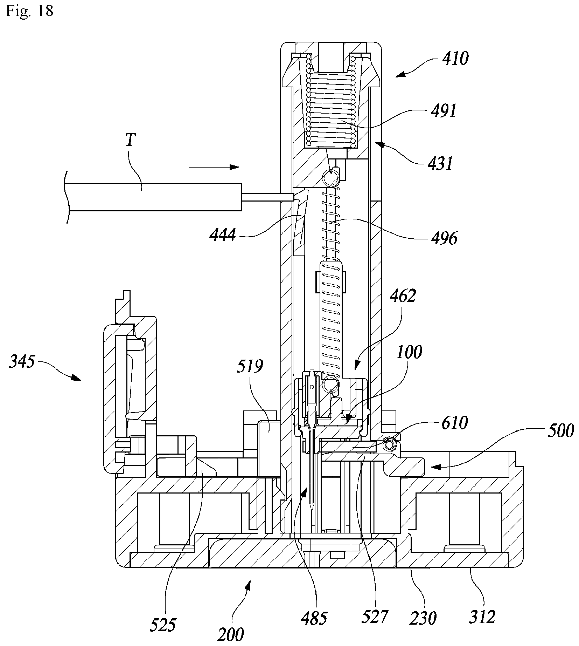

Then, an operator may switch the insertion unit (430) into an operable state by removing the safety pin (P) and manipulating the clamping hook (444) to release the temporary fixing state of the shuttle (431) as shown in FIGS. 17 and 18. An operator may disengage the clamping hook (444) from the column member detent (423) of the column member (410) by simply pressing the clamping hook (444) into the column member (410) using a simple tool (T). When an operator presses the clamping hook (444) into the column member (410), the clamping hook (444) is released from the column member detent (423) of the column member (410) and the shuttle (431) is slightly pushed by the elastic member (491) of the shuttle driver (490), so that the clamping hook (444) can be maintained away from the column member detent (423).

As such, the applicator assembly (10) according to an embodiment of the present disclosure can be simply assembled to the applicator body (300) by forming a single assembly in which the shuttle (431), a needle assembly (460), a shuttle driver (490), and a needle release driver (495), which constitute the insertion unit (430), together with the column member (410). Therefore, installation of the insertion unit (430) is easy, the task of installing the insertion unit (430) on the applicator body (300) can be partially automated, and manufacturing time can be reduced. Additionally, the sensor unit (100) can be simply installed on the applicator body (300) in a state of being coupled to the insertion unit (430) from the outside of the applicator body (300).

Additionally, the applicator assembly (10) according to an embodiment of the present disclosure may be coupled to the applicator body (300) in a locked state to prevent accidental operation of the insertion unit (430). And the insertion unit (430) can be converted to an operable state by simple manipulation after being coupled to the applicator body (300). Therefore, after installing the insertion unit (430) on the applicator body (300), there is no need for cumbersome follow-up work to convert the shuttle (431) and carrier (462) of the insertion unit (430) into an operable state.

As another exemplary embodiment, the insertion unit (430) is coupled to the applicator body (300) through the column member (410), and then the shuttle (431) and the carrier (462) can be moved to an operable position by a user.

The stopper member (500) is positioned to move linearly on the stage (333) of the middle frame (330). The stopper member (500) is installed to move in a direction intersecting the direction in which the sensor unit (100) moves from the first position to the second position. In the drawing, the stopper member (500) is shown to move in a direction perpendicular to the direction of movement of the sensor unit (100), but the movement direction of the stopper member (500) may be changed in various ways. The stopper member (500) may move from a third position, which supports the shuttle (431) to be positioned in the first position, to a fourth position where it disengages from the shuttle (431).

The stopper member (500) may restrict the movement of the shuttle (431) placed in the first position, by contacting the shuttle protrusion (435) of the shuttle (431), which is located in the first position, at the third position, and move away from the shuttle protrusion (435) when moving to the fourth position, so that the shuttle (431) can move to the second position. In addition, the stopper member (500) may restrict the movement of removing member (610) so that the removing member (610) of the removing unit (600) maintains the coupled position with the adhesive layer (155) of the sensor unit (100), which is located in the first position, at the third position.

The stopper member (500) can move by receiving a moving force from the stopper member driver (540). The stopper member driver (540) provides a moving force to the stopper member (500) to move the stopper member (500) from the third position to the fourth position. The stopper member driver (540) includes an elastic member (541) that applies elastic force in a direction that deflects the stopper member (500) toward the fourth position. The elastic member (541) may have one end coupled to the fixing protrusion (337) of the middle frame (330) and the other end in contact with the stopper member (500) to apply an elastic force to the stopper member (500).

The stopper member driver (540) may be changed into various other forms that can provide a moving force to the stopper member (500) in addition to the configuration including the elastic member (541) in the form of a coil spring.

The stopper member (500) includes a stopper member body (510), a supporting portion (519) for supporting the shuttle (431), a stopper member fixing portion (521) capable of engaging with the applicator body (300), a stopper member locking portion (524) for fixing the shuttle (431), which has reached the second position, to the second position, and a a stopper portion (527) capable of supporting the removing member (610).

A stopper member opening (512) and a stopper member groove (513) are formed in the stopper member body (510) to penetrate the stopper member body (510) in the thickness direction. A fixing protrusion (514) to which the other end of the elastic member (541) is coupled is provided in the stopper member groove (513). A cover portion (515) is provided above the stopper member groove (513) to cover the elastic member (541) placed in the stopper member groove (513).

A rail portion (517) is provided on one surface of the stopper member body (510) facing the stage (333) of the middle frame (330). The rail portion (517) may engage with the guide rail (336) of the middle frame (330) and slide along the guide rail (336).

The supporting portion (519) is placed at the edge of the stopper member opening (512) so as to protrude from the stopper member body (510). The supporting portion (519) may support the shuttle protrusion (435) of the shuttle (431). When the stopper member (500) is positioned in the third position, the supporting portion (519) may support the shuttle protrusion (435) of the shuttle (431) to restrict the movement of the shuttle (431) positioned in the first position. And when the stopper member (500) moves to the fourth position, the supporting portion (519) deviates from the shuttle protrusion (435), so that the shuttle (431) can move to the second position. In addition to the shape that protrudes from the stopper member body (510), the supporting portion (519) can be changed into various other shapes that can contact or engage with the shuttle protrusion (435) or other parts of the shuttle (431).

The stopper member fixing portion (521) may restrict the movement of the stopper member (500) by engaging with one side of the applicator body (300) so that the stopper member (500) maintains the elastically deformed state of the elastic member (541). The stopper member fixing portion (521) includes a pair of fixing hooks (522) that can be engaged with the support fixture (339) of the middle frame (330). The fixing hook (522) may be disengaged from the support fixture (339) by the operating member (345). The fixing hook (522) may be inserted into the support fixture opening (342) of the support fixture (339) and engaged with one side of the support fixture (339). At this time, the stopper member (500) remains fixed in the third position. When the operating member (345) is pressed by a user and advances toward the fixing hook (522), the pressing protrusion (348) of the operating member (345) presses the fixing hook 522. At this time, the fixing hook (522) is biased to be disengaged from the support fixture (339). When the fixing hook (522) is disengaged from the support fixture (339) by the operating member (345), the stopper member (500) is moved to the fourth position by the elastic force of the elastic member (541)

The configuration of the stopper member fixing portion (521) for restraining the movement of the stopper member (500) so that the stopper member (500) maintains the elastically deformed position of the elastic member (541) is not limited to that shown and can be changed in various ways. As another exemplary embodiment, the stopper member fixing portion may be provided on the applicator body (300) so as to engage with the stopper member (500) located at the third position.

The stopper member locking portion (524) may lock the shuttle (431) in the second position by engaging with the shuttle locking portion (446) of the shuttle (431) that has moved to the second position. The stopper member locking portion (524) includes a locking protrusion (525) that can be inserted into the locking groove (447) of the shuttle locking portion (446). The locking protrusion (525) is provided on one side of the inner surface of the stopper member body (510) so as to protrude into the stopper member opening (512).

As shown in (a) of FIG. 20, when the stopper member (500) is located in the third position, the locking protrusion (525) is placed at a position corresponding to the opening (429) of the column member (410). At this time, the supporting portion (519) of the stopper member (500) supports the shuttle protrusion (435) of the shuttle (431), so that the shuttle (431) maintains in the first position.

Meanwhile, as shown in (b) of FIG. 20, when the stopper member (500) moves to the fourth position, the shuttle protrusion (435) of the shuttle (431) moves away from the supporting portion (519) of the stopper member (500), and the shuttle (431) moves to the second position. Then, the locking protrusion (525) passes through the opening (429) of the column member (410) and is inserted into the locking groove (447) of the shuttle (431). Since the stopper member (500) is engaged with the shuttle (431), the stopper member (500) may restrict the movement of the shuttle (431) so that the shuttle (431) that has moved to the second position cannot move. At this time, the shuttle (431) cannot return to the first position, and reuse of the applicator (30) may be prevented.

The stopper member locking portion (524) and the shuttle locking portion (446) are not limited to the configuration shown and may be changed in various ways.

The stopper portion (527) is connected to one side of the stopper member body (510) and is placed in the stopper member opening (512). The stopper portion (527) is in contact with the removing member (610) to maintain the position in which the removing member (610) is coupled to the protective sheet (160) of the sensor unit (100) located in the first position and restrains the movement of removing member (610)

The stopper member (500) may restrict the movements of both the shuttle (431) and the removing member (610) in the third position. While moving from the third position to the fourth position, the stopper member (500) may first release the binding force on the removing member (610) and then release the binding force on the shuttle (431). Accordingly, the separation operation of the protective sheet (160) is completed in a state in which the sensor unit (100) is located in the first position, and then the shuttle (431) moves so that the sensor unit (100) can be moved to the second position. In some cases, a configuration in which the shuttle (431) starts to move toward the second position during the separation operation of the protective sheet (160) by the removing member (610) may be possible.

In this way, the stopper member (500) can be moved by the operating member (345) to operate the removing unit (600) and the insertion unit (430) sequentially or simultaneously.

The removing unit (600) is installed on the applicator body (300) to separate the protective sheet (160) of the sensor unit (100) from the adhesive layer (155) before the sensor unit (100) reaches the second position and is coupled to the base unit (200). The removing unit (600) includes a removing member (610) rotatably installed on the applicator body (300) to be coupled to the protective sheet (160), and a removing member driver (620) that provides rotational force to the removing member (610).

The removing member (610) may be installed on the applicator body (300) to rotate about a central rotation axis perpendicular to the moving direction of the sensor unit (100). The removing member (610) is provided with a hinge portion (611). The removing member (610) can rotate around the hinge portion (611) because the hinge portion (611) is rotatably coupled to the support (318) of the base frame (310). A removing member groove (613) corresponding to the protective sheet opening (162) of the protective sheet (160) is formed on one side of the removing member (610). When the removing member (610) is coupled to the protective sheet (160) of the sensor unit (100) located in the first position, the boss (127) of the sensor unit housing (120) may be located in the removing member groove (613). The removing member (610) may be coupled to the protective sheet (160) through the adhesive member (164) provided on the surface of the protective sheet (160). Various other methods other than using the adhesive member (164) may be used to connect the removing member (610) and the protective sheet (160).

The removing member driver (620) includes a removing elastic member (621) that is coupled to the removing member (610) and applies an elastic force to the removing member (610). The removing elastic member (621) is in the form of a torsion spring wound around the hinge portion (611). One side of the removing elastic member (621) is in contact with the applicator body (300) so as to provide the removing member (610) with an elastic force that can rotate the removing member (610).

When the removing member (610) is supported by the stopper portion (527) of the stopper member (500) and placed substantially parallel to the protective sheet (160) of the sensor unit (100) located in the first position, the protective sheet (160) may be coupled with the removing member (610) in a state of covering the adhesive layer (155).

Meanwhile, when the stopper member (500) moves toward the fourth position, the stopper portion (527) moves away from the removing member (610), and the binding force of the removing member (610) by the stopper portion (527) may be released. At this time, the removing member (610) may be rotated by the elastic force of the removing elastic member (621), thereby separating the protective sheet (160) from the adhesive layer (155).

The configuration of the removing member (610) or the removing member driver (620) which constitutes the removing unit (600) is not limited to that shown and can be changed in various ways.

The removing unit (600) may complete the separation operation of the protective sheet (160) before the sensor unit (100) moves from the first position. As described above, as the stopper member (500) moves from the third position to the fourth position, the restraining force on the removing member (610) may first be released and then the binding force on the shuttle (431) may be released. Therefore, it is possible for the removing member (610) to complete the task of separating the protective sheet (160) before the shuttle (431) moves. In this way, by completing the separation operation of the protective sheet (160) before the sensor unit (100) moves from the first position, a problem in which the sensor unit (100) reaches the base unit (200) without separating the protective sheet (160) may be prevented.

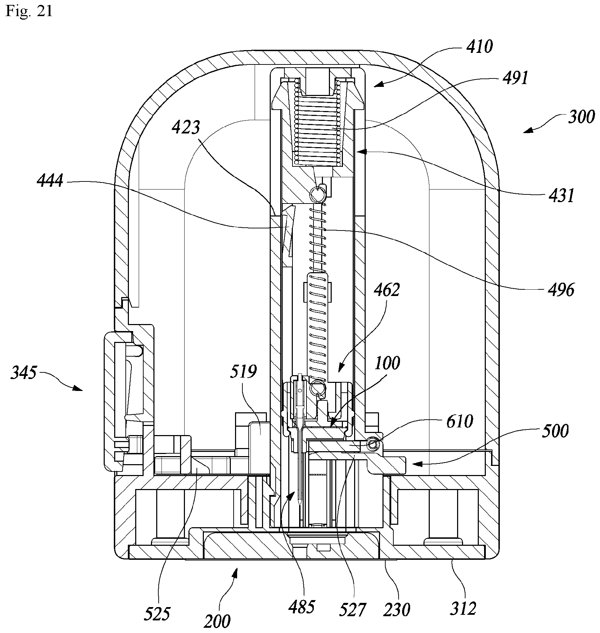

Hereinafter, with reference to FIGS. 21 to 23, a process of attaching the body attachable unit (20) to the skin of a user using the applicator (30) according to an embodiment of the present disclosure will be described.

First, as shown in FIG. 21, in order to attach the body attachable unit (20) to the skin of a user, The bottom portion (312) of the applicator (30) is positioned on the skin of a user so that the base unit (200) is attached to the skin of a user by the adhesive portion (230). Before the operating member (345) is manipulated, the sensor unit (100) coupled to the carrier (462) is positioned in the first position in a state in which the protective sheet (160) is covering the adhesive layer (155). At this time, the supporting portion (519) of the stopper member (500) supports the shuttle (431) to maintain the position where the shuttle (431) is stopped at the first position. And the stopper portion (527) of the stopper member (500) supports the removing member (610), so that the removing member (610) maintains the position coupled to the protective sheet (160).

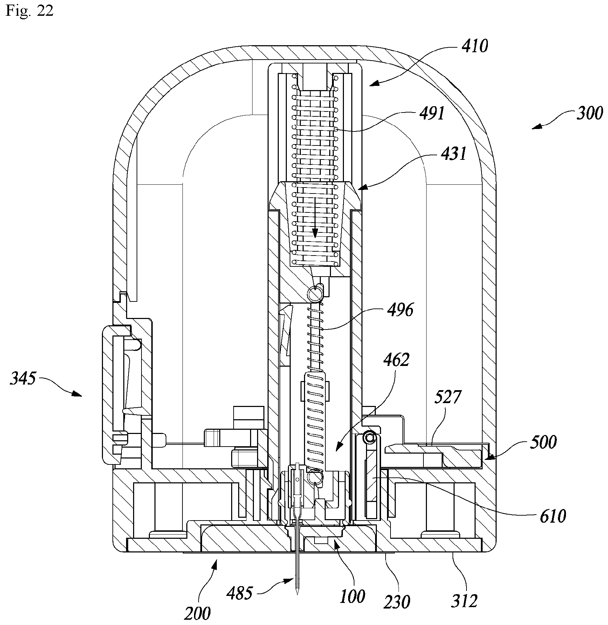

Thereafter, when a user presses the operating member (345), the operating member (345) disengages the stopper member fixing portion (521) of the stopper member (500) from the applicator body (300). At this time, as shown in FIG. 22, the stopper member (500) is moved from the third position to the fourth position by the stopper member driver (540). As the stopper member (500) moves from the third position to the fourth position, the binding force on the removing member (610) is released first, and then the binding force on the shuttle (431) is sequentially released. Accordingly, after the removing member (610) rotates to separate the protective sheet (160) from the adhesive layer (155) of the sensor unit (100), the shuttle (431) moves to the second position. As the shuttle (431) moves to the second position, the needle (485) and the sensor (110) are inserted into the skin of a user, and the sensor unit (100) is attached to the base unit (200) by the adhesive layer (155) so that the attachable unit (20) is assembled. And the locking protrusion (525) of the stopper member (500) is inserted into the locking groove (447) of the shuttle (431) that has moved to the second position. Accordingly, the movement of the shuttle (431) is restricted and the shuttle (431) cannot return to the first position.

Additionally, when the shuttle (431) reaches the second position, the shuttle (431) moves the locking hook (360) so that the locking hook (360) is disengaged from the base unit (200), and the trigger (474) of the carrier (462) comes into contact with the release portion (420) of the column member (410). At this time, the carrier wing (471) of the carrier (462) is elastically deformed, thereby disengaging the carrier latch (477) from the shuttle detent (438).

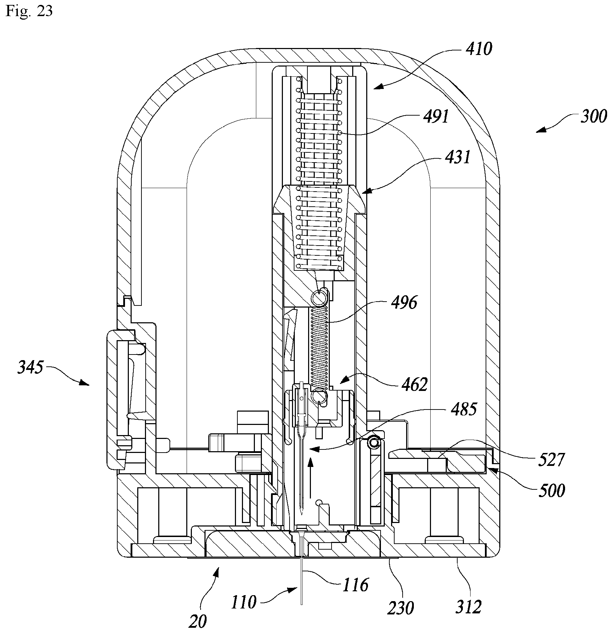

When the carrier (462) is disengaged from the shuttle (431), as shown in FIG. 23, the carrier (462) is moved in a direction away from the skin of a user by the needle release driver (495). At this time, the needle (485) comes out of the skin of a user and the body attachable unit (20) remains attached to the skin of a user by the adhesive portion (230).

After the body attachable unit (20) is attached to the skin of a user, the applicator (30) is separated from the body attachable unit (20), and the body attachable unit (20) may measure the biometric information of a user and transmit the measurement information to an external terminal (5), etc.

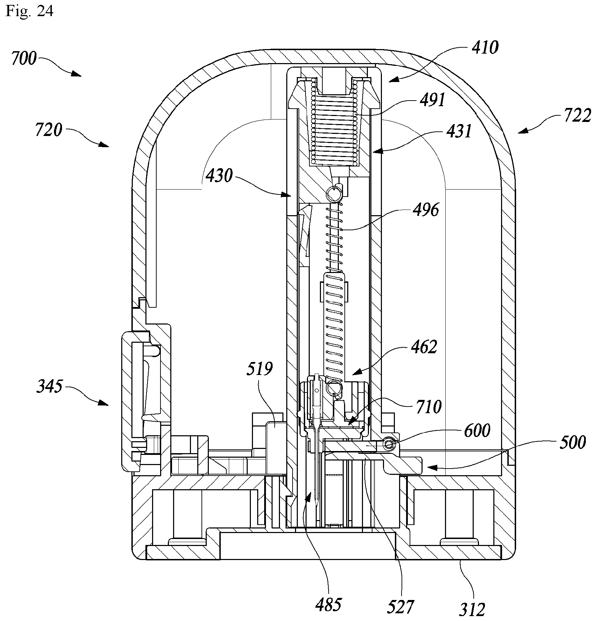

Meanwhile, FIG. 24 shows an applicator assembly according to another embodiment of the present disclosure.

The applicator assembly (700) shown in FIG. 24 includes a sensor unit (710) including a sensor (110) that is inserted into the skin of a user and measures biometric information, and an applicator (720) on which the sensor unit (710) is detachably mounted to insert the sensor (110) into the skin of a user. The sensor unit (710) can be moved from the first position to the second position by the insertion unit (430) which is installed in the applicator body (722) by the column member (410). The insertion unit (430) is mounted on the column member (410) and can be assembled to the applicator body (722) with the column member (410) by coupling the column member (410) to the applicator body (722).

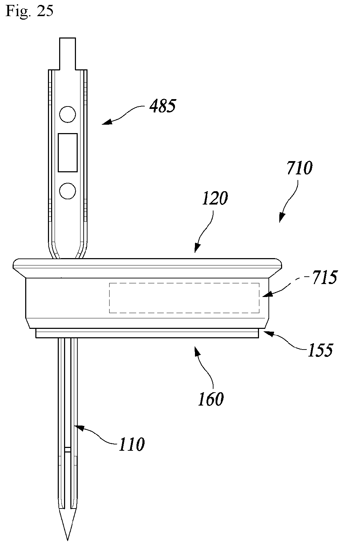

As shown in FIG. 25, the sensor unit (710) includes a sensor (110) inserted into the skin of a user, a sensor unit housing (120) to which the sensor (110) is coupled, an adhesive layer (155) provided on the sensor unit housing (120), a protective sheet (160) covering the adhesive layer (155), and an electronic component (715). The electronic component (715) includes a circuit board electrically connected to the sensor (110), a process chip installed on the circuit board to convert biometric information measured by the sensor (110) into an electrical signal, and a communication chip for communication with the outside, and batteries, etc. The sensor unit (710) may be attached to the skin of a user by moving from the first position to the second position in a state of being coupled with the needle (485).

The applicator (720) can remove the protective sheet (160) from the sensor unit (710) using the removing unit (600) and move the sensor unit (710) from the first position to the second position using the inserting unit (430). After the shuttle (431) of the insertion unit (430) moves to the second position, and moves the sensor unit (710) to the second position, the stopper member (500) engages with the shuttle (431) and restricts the movement of the shuttle (431) to prevent it from returning to the first position.

The sensor unit (710) may be inserted into the skin of a user in the second position and attached to the skin of a user by the adhesive layer (155). After the sensor unit (710) is attached to the skin of a user, the needle (485) is separated from the skin, and the sensor unit (710) is separated from the applicator (720) and can measure the user's biometric information.

The applicator assembly (700) according to this embodiment can operate without the need for a separate base unit because the sensor unit (710) can be directly attached to the skin of a user and has signal processing and communication functions. That is, the sensor unit (710) can measure biometric information, and transmit the measured biometric information to an external terminal 5, etc. by being attached to the skin of a user.

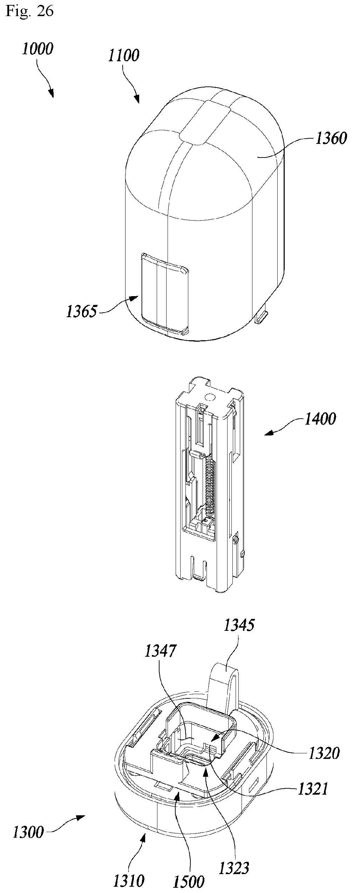

Meanwhile, FIG. 26 shows an exploded view of a partial configuration of an applicator according to another embodiment of the present disclosure.

The applicator assembly (1000) shown in FIG. 26 includes a sensor unit (100) having a sensor (110) that is inserted into the skin of a user and measures biometric information, a base unit (200) that forms a body attachable unit with the sensor unit (100), and an applicator (1100) for attaching the body attachable unit to the skin of a user. The sensor unit (100) and the base unit (200) may be installed on the applicator (1100) in a separated state and form a body attachable unit by being coupled to each other by the applicator (1100).

The applicator (1100) includes an applicator body (1300) to which the base unit (200) is detachably coupled, an operating member (1365) installed on the applicator body (1300) for user manipulation, an insertion unit assembly (1400) including an insertion unit (1440) for inserting a sensor (110) into the skin of a user, and a stopper member (1500) movably installed on the applicator body (1300).

The applicator body (1300) includes a base frame (1310) and a top case (1360) that covers the upper part of the base frame (1310).

The base frame (1310) has a bottom portion (1312) that can be in contact with the skin of a user. The bottom portion (1312) of the base frame (1310) is provided with a recess (1313) on which the base unit (200) is mounted. A stage (1331) supporting the stopper member (1500) is provided on the upper part of the base frame (1310). An applicator body groove (1347) into which the insertion unit assembly (1400) is inserted is provided in the middle of the stage (1331). An applicator body snap portion (1320) is provided inside the applicator body groove (1347) to secure the column member (1410) of the insertion unit assembly (1400). The applicator body snap portion (1320) includes a snap groove (1321) with which the column member (1410) can be engaged. The applicator body snap portion (1320) forms an applicator body coupling portion (1323) for fixing the column member (1410) to the applicator body (1300) with the applicator body groove (1347).

A return member (1345) that can return the stopper member (1500) to its original position is installed on one side of the base frame (1310). The return member (1345) can be elastically deformed by the stopper member (1500) and can return the stopper member (1500) which is moved by the operating member (1365) to its original state. The return member (1345) is elastically deformed by the stopper member (1500) moved forward by the operating member (1365) and can apply an elastic force to the stopper member (1500) in a retreating direction.

An operating member (1365) for user operation is installed in the top case (1360). The operating member (1365) includes a button portion (1366) and a pressing protrusion (1367) protruding from the button portion (1366). When the operating member (1365) is pressed into the applicator body (1300) by a user, the pressing protrusion (1367) pushes the stopper member (1500) toward the return member (1345), thereby moving the stopper member (1500).

The insertion unit assembly (1400) includes a column member (1410) fixed to the applicator body (1300), and an insertion unit (1440) that is supported on the column member (1410) to be coupled to the sensor unit (100) and moves the sensor unit (100) from the first position to the second position. The insertion unit (1440) is coupled to the applicator body (1300) through the column member (1410). The insertion unit (1440) includes a shuttle (1441) movably installed inside the column member (1410) and a needle assembly (1460) that can move with the shuttle (1441). The shuttle (1441) may be coupled with the sensor unit (100) and move from the first position to the second position inside the applicator body (1300). The needle assembly (1460) includes a carrier (1462) capable of relative movement with respect to the shuttle (1441) and a needle (1485) that can be inserted into the skin of a user by being coupled to the carrier (1462).

The column member (1410) supports the insertion unit (1440) by being fixed to the applicator body (1300). The column member (1410) includes a column member body (1411). The column member body (1411) has a space inside to accommodate the insertion unit (1440), and has a column member opening (1412) open to the outside at one end. One end of the column member body (1411) is provided with a coupling end portion (1435) that is inserted into the applicator body groove (1347) of the applicator body (1300). The coupling end portion (1435) includes a plurality of column member legs (1413). The column member leg (1413) may be provided in an elastically deformable form. Additionally, one end of the column member body (1411) is provided with a column member snap portion (1414) that can engage with the applicator body snap portion (1320) of the applicator body (1300). The column member snap portion (1414), together with the coupling end portion (1435), constitutes a column member coupling portion (1434) coupled to the applicator body coupling portion (1323). The column member (1410) can be more firmly fixed to the applicator body (1300) by engaging the column member snap portion (1414) with the applicator body snap portion (1320). The column member snap portion (1414) includes an elastically deformable snap arm (1415). The snap arm (1415) is provided with a snap hook (1416) that engages the snap groove (1321) of the applicator body snap portion (1320). When the column member (1410) is inserted into the applicator body groove (1347), a portion of the snap hook (1416) is inserted into the snap groove (1321).

In this way, the column member (1410) can be simply coupled to the applicator body (1300) by inserting one end into the applicator body groove (1347) of the applicator body (1300).

The coupling method of the applicator body (1300) and the column member (1410) is not limited to that shown and may be changed in various ways. As another exemplary embodiment, the position or number of applicator body snap portion (1320) provided in the applicator body (1300) and the position or number of column member snap portion (1414) provided in the column member (1410) can be changed in various ways. As another exemplary embodiment, the column member (1410) can be coupled to the applicator body (1300) by a forced press-fit into the applicator body groove (1347). As another exemplary embodiment, the column member (1410) may be coupled to the applicator body (1300) in such a way that a portion of the applicator body (1300) is inserted into the column member opening (1412) of the column member (1410). Additionally, the applicator body coupling portion (1323) and the column member coupling portion (1434) may be changed into various other configurations that can be engaged with each other other than the configuration shown.

Slits (1418) are formed on both sides of the column member body (1411) in a direction parallel to the moving direction of the shuttle (1441). A release portion (1419) is provided in the middle portion of the slit (1418). The release portion (1419) is a portion of the carrier (1462) that can come into contact while the carrier (1462) moves toward the base unit (200). The carrier (1462) is disengaged from the shuttle (1441) by action of the release portion (1419), and can relatively move with respect to the shuttle (1441).

Additionally, guide grooves (1420) are formed on both sides of the column member body (1411) in a direction parallel to the moving direction of the shuttle (1441). The shuttle hook (1443) of the shuttle (1441) is placed in the guide groove (1420), so that the shuttle hook (1443) can move along the guide groove (1420). A column member detent (1421) is provided at the end of the guide groove (1420) to limit the moving distance of the shuttle (1441). When the shuttle (1441) moves from the first position with the sensor unit (100) and reaches the second position, the shuttle hook (1443) comes into contact with the column member detent (1421) so that the shuttle (1441) can stop at the second position.

A clamping portion (1424) and a blocking portion (1429) that can restrict the movement of the shuttle (1441) are provided on both sides of the column member body (1411).

The clamping portion (1424) may engage with the shuttle (1441) and may be used to temporarily fix the shuttle (1441) to the column member (1410) during the process of assembling the insertion unit assembly (1400). The clamping portion (1424) includes a clamping portion arm (1425) provided with a clamping portion detent (1426), and a knob (1427) connected to the clamping portion arm (1425) for user operation. The clamping portion detent (1426) may be formed to protrude inward from the column member body (1411), or may be formed to engage with one side of the shuttle (1441). The clamping portion arm (1425) is elastically deformably connected to the column member body (1411). When a user pulls the knob (1427), the clamping portion arm (1425) may be elastically deformed.

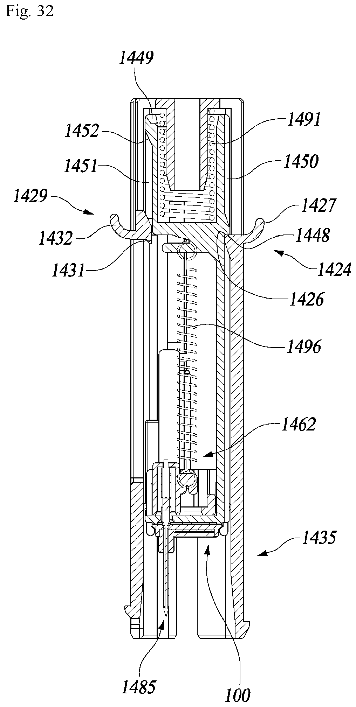

The insertion unit assembly (1400) may be coupled to the applicator body (1300) in a state in which the shuttle (1441) is temporarily fixed to the column member (1410) by the clamping portion (1424). While the shuttle (1441) is temporarily fixed to the column member (1410) by the clamping portion (1424), the shuttle (1441) cannot move. As shown in FIG. 32, after the shuttle (1441) is inserted into the column member (1410), when the clamping portion detent (1426) of the clamping portion (1424) is engaged with the shuttle latch (1448) of the shuttle (1441), the shuttle (1441) is temporarily fixed to the column member (1410). Since the shuttle latch (1448) is engaged with the clamping portion detent (1426), the shuttle (1441) cannot relatively move with respect to the column member (1410).

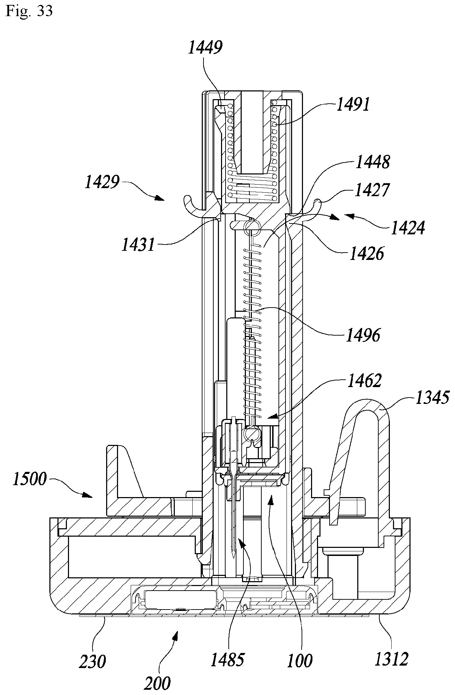

After coupling the insertion unit assembly (1400) to the applicator body (1300) as shown in FIG. 33, a user pulls the knob (1427) to elastically deform the clamping portion arm (1425) thereby releasing the clamping portion detent (1426) from the shuttle latch (1448).

A variety of other methods may be used to temporarily fix the shuttle (1441) to the column member (1410) in addition to using the clamping portion (1424) provided on the column member (1410) to be elastically deformable.

The blocking portion (1429) is for fixing the shuttle (1441), which has reached the second position, to the second position. The blocking portion (1429) includes a blocking portion arm (1430) provided with a blocking portion detent (1431) and a knob (1432) connected to the blocking portion arm (1430) for user manipulation. The blocking portion detent (1431) may have a shape that protrudes inward from the column member body (1411), or may have another shape that can be in contact with one side of the shuttle (1441). The blocking portion arm (1430) is elastically deformably connected to the column member body (1411).

After the applicator (1100) operates to insert the sensor (110) into the skin of a user, the applicator (1100) needs to be separated from the body attachable unit and disposed of. The blocking portion (1429) may prevent reuse of the applicator (1100) by restricting the movement of the shuttle (1441) after the shuttle (1441) moves to the second position. After the shuttle (1441) moves to the second position, the blocking portion detent (1431) comes into contact with one side of the shuttle (1441), thereby preventing the shuttle (1441) from moving to the first position. And after the applicator (1100) is used, the shuttle (1441) is fixed at the second position, so the applicator (1100) cannot be reused.

The shuttle (1441) is installed to be linearly movable inside the column member (1410). The shuttle (1441) includes a shuttle body (1442) with a space inside to accommodate the needle assembly (1460). A shuttle hook (1443) is provided on one side of the shuttle body (1442) to limit the movement range of the shuttle (1441). A shuttle protrusion (1444) is provided on the other side of the shuttle (1441). The shuttle protrusion (1444) protrudes from the shuttle body (1442) so as to contact the stopper member (1500). The shuttle (1441) is fixed in the first position when the shuttle protrusion (1444) comes into contact with the stopper member (1500), and can move to the second position when the shuttle protrusion (1444) leaves the stopper member (1500).

A pair of slits (1445) are formed on both sides of the shuttle body (1442) in a direction parallel to the moving direction of the shuttle (1441). A shuttle detent (1446) to which the carrier latch (1477) of the carrier (1462) can be engaged is provided at a middle portion of the slit (1445). In a state in which the carrier latch (1477) is engaged with the shuttle detent (1446), the carrier (1462) moves with the shuttle (1441) and cannot relatively move with respect to the shuttle (1441). And when the carrier latch (1477) is disengaged from the shuttle detent (1446), the carrier (1462) can relatively move with respect to the shuttle (1441).

On both sides of the shuttle body (1442), a shuttle latch (1448) that can engage with the clamping portion (1424) of the column member (1410), and a detent portion (1449) that can contact to the blocking portion (1429) are provided.

When the shuttle latch (1448) engages with the clamping portion detent (1426) of the clamping portion (1424), the shuttle (1441) may be temporarily fixed to the column member (1410). When the temporary fixing state by the clamping portion (1424) is released, the clamping portion detent (1426) is inserted into the guide groove (1450) formed in the shuttle body (1442) to be adjacent to the shuttle latch (1448). When the shuttle (1441) moves from the first position to the second position, the clamping portion detent (1426) may relatively move with respect to the shuttle (1441) in the guide groove 1450. The shape or location of the shuttle latch (1448) is not limited to that shown and may be changed in various ways.

The detent portion (1449) is provided at the end of the shuttle body (1442) so as to contact the blocking portion detent (1431) of the blocking portion (1429). After the shuttle (1441) moves to the second position, the detent portion (1449) is placed to face the blocking portion detent (1431), so that the shuttle (1441) cannot move to the first position. The blocking portion detent (1431) is inserted into the guide groove (1451) formed in the shuttle body (1442) and can relatively move with respect to the shuttle (1441). The guide groove (1451) is formed in a direction parallel to the moving direction of the shuttle (1441). A shuttle inclined portion (1452) is positioned on one side of the guide groove (1451). The shuttle inclined portion (1452) is provided in an inclined form with respect to the moving direction of the shuttle (1441). When the shuttle (1441) moves to the second position, the blocking portion detent (1431) relatively moves with respect to the guide groove (1451), and when it reaches the shuttle inclined portion (1452), the blocking portion arm (1430) may be elastically deformed. As the blocking portion arm (1430) is elastically deformed, the blocking portion detent (1431) can smoothly pass through the shuttle inclined portion (1452) and reach a position facing the detent portion (1449). The shape or location of the detent portion (1449) is not limited to that shown and may be changed in various ways.

A shuttle bottom portion (1453) in contact with the sensor unit (100) is provided at one end of the shuttle body (1442). The shuttle bottom portion (1453) is provided with a shuttle through-hole (1454) into which the needle (1485) is inserted, and a pressing protrusion (1455) inserted into the sensor unit groove (138) of the sensor unit (100).

Additionally, shuttle (1441) includes shuttle opening (1458). Shuttle opening (1458) is formed on one side of the shuttle body (1442) to open outward. During the process of assembling the insertion unit assembly (1400), the needle assembly (1460) may be installed inside the shuttle body (1442) through the shuttle opening (1458).

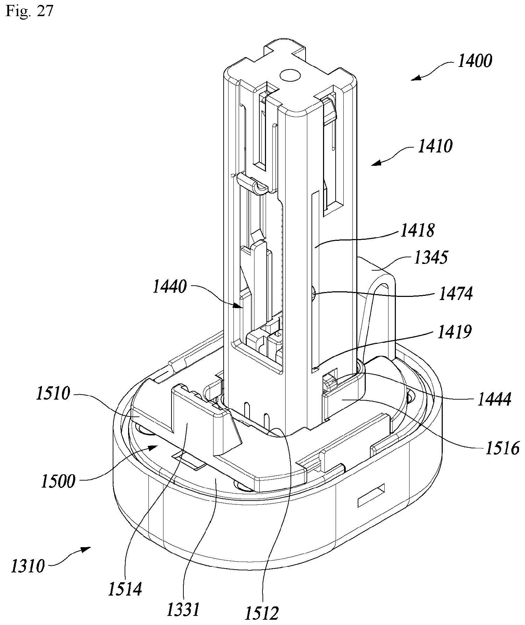

The shuttle (1441) moves by receiving movement force from the shuttle driver (1490). The shuttle driver (1490) includes an elastic member (1491) that applies elastic force to the shuttle (1441) in the direction of moving from the first position to the second position. As shown in FIG. 32, the shuttle (1441) may maintain a state in which the elastic member (1491) is elastically deformed and temporarily fixed to the column member (1410) by the clamping portion (1424) before the column member (1410) is coupled to the applicator body (1300). And as shown in FIG. 27, after the insertion unit assembly (1400) is coupled to the applicator body (1300), as the clamping portion (1424) is disengaged from the shuttle latch (1448) and the shuttle protrusion (1444) comes in contact to the stopper member (1500), the shuttle (1441) can be maintained in a stopped state in the first position by the stopper member (1500).

The needle assembly (1460) includes a carrier (1462) to which the sensor unit (100) is detachably coupled, and a needle (1485) coupled to the carrier (1462) to penetrate the sensor unit (100). The needle (1485) includes a needle body (1486) that can be inserted into the skin of a user, and a needle head (1487) coupled to the carrier (1462).

The carrier (1462) includes a carrier body (1463) to which the needle (1485) is coupled, an elastically deformable carrier wing (1471) extending from the carrier body (1463), and a grip arm (1479) connected to the side of the carrier body (1463). An insertion groove (1465) into which a portion of the needle (1485) is inserted is formed on one side of the carrier body (1463). The carrier wing (1471) is elastically deformably connected to the carrier body (1463). Carrier wing (1471) includes trigger (1474) and carrier latch (1477). The trigger (1474) may be inserted into the slit (1445) of the shuttle (1441) and the slit (1418) of the column member (1410), and may contact the release portion (1419) of the column member (1410). The carrier latch (1477) may engage with shuttle detent (1446) of shuttle (1441). In a state in which the carrier latch (1477) engages with the shuttle detent (1446), the carrier (1462) can maintain the elastic member (1496) in an elastically deformed state and cannot move in the direction in which the elastic force of the elastic member (1496) acts. And when the trigger (1474) contacts the release portion (1419) and the carrier wing (1471) is elastically deformed, the carrier latch (1477) may deviate from the shuttle detent (1446) and the carrier (1462) can move. The grip arm (1479) is provided to be elastically deformable on the side of the carrier body (1463) and can engage with the sensor unit (100). The grip arm (1479) is provided with a grip protrusion (1480) engaged with the sensor unit housing (120) and a protrusion (1481) in contact with the inner wall of the column member (1410).

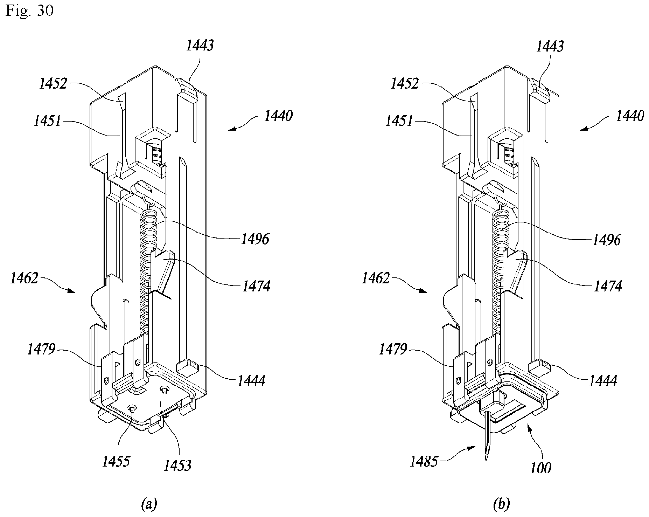

As shown in (a) of FIG. 30, the carrier (1462) is coupled to the shuttle (1441) so that the carrier body (1463) is located inside the shuttle (1441) and the end of the grip arm (1479) protrudes from the shuttle (1441). And, as shown in (b) of FIG. 30, the carrier (1462) can fix the sensor unit (100) to the shuttle (1441) in such a way that a pair of grip arms (1479) engage with the sensor unit (100).

Carrier (1462) can relatively move with respect to shuttle (1441) by needle release driver (1495). The needle release driver (1495) includes an elastic member (1496) that provides a moving force to the carrier (1462).

As shown in FIG. 32, the insertion unit assembly (1400) may be coupled to the applicator body (1300) in a state in which the elastic member (1491) of the shuttle driver (1490) for moving the shuttle (1441) and the elastic member (1496) of the needle release driver (1495) for moving the needle assembly (1460) are elastically deformed. Additionally, the insertion unit assembly (1400) may be coupled to the applicator body (1300) while being coupled to the sensor unit (100). As described above, in the process in which the insertion unit assembly (1400) is coupled to the applicator body (1300), the shuttle (1441) may be temporarily fixed to the column member (1410) by the clamping portion (1424), thereby preventing the accidental movement of the shuttle (1441).

As shown in FIG. 33, after the insertion unit assembly (1400) is coupled to the applicator body (1300), the clamping portion (1424) is manipulated to release the restraining force on the shuttle (1441), so that the insertion unit (1440) may be brought into an operable state by the operating member (1365). The clamping portion (1424) can be simply disengaged from the shuttle latch (1448) of the shuttle (1441). For example, when a user pulls the knob (1427) to elastically deform the clamping portion arm (1425), the clamping portion detent (1426) may be released from the shuttle latch (1448). At this time, the shuttle protrusion (1444) of the shuttle (1441) comes into contact with the stopper member (1500), so that the shuttle (1441) can remain stopped in the first position in a state in which the elastic member (1491) elastically deformed.

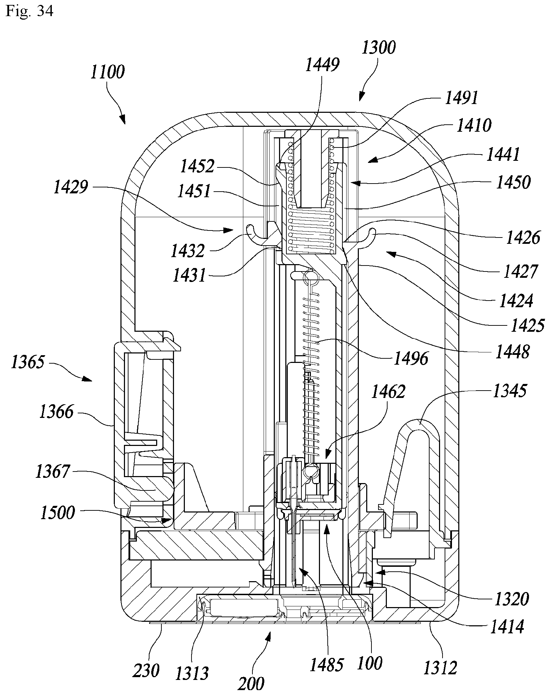

As shown in Figure 34, when the clamping portion detent (1426) leaves the shuttle latch (1448), the shuttle (1441) is slightly pushed by the elastic member (1491) of the shuttle driver (1490), thereby causing the clamping portion detent (1426) is located in the guide groove (1450) of the shuttle (1441) and can be maintained out of the shuttle latch (1448).

As such, the applicator assembly (1000) according to this embodiment can be simply assembled to the applicator body (1300) by forming a single assembly in which the shuttle (1441), a needle assembly (1460), a shuttle driver (1490), and a needle release driver (1495), which constitute the insertion unit (1440), together with the column member (1410). Therefore, installation of the insertion unit (1440) is easy, and manufacturing time can be reduced.

The stopper member (1500) is positioned to move linearly on the stage (1331) of the middle frame (1330). The stopper member (1500) may move from a third position, which supports the shuttle (431) to be positioned in the first position, to a fourth position, where it is disengaged from the shuttle (1441). The stopper member (1500) is in contact with the shuttle protrusion (1444) of the shuttle (1441), which is located in the first position, in the third position to constrain the movement of the shuttle (1441) placed in the first position, and when moving to the fourth position, the shuttle (1441) may move away from the shuttle protrusion (1444) to move to a second position.

The stopper member (1500) includes a stopper member body (1510) and a supporting portion (1516) for supporting the shuttle (1441). A stopper member opening (1512) into which the insertion unit assembly (1400) is inserted is provided in the middle portion of the stopper member body (1510). At one end of the stopper member body (1510), a bracket portion (1514), which the operating member (1365) may contact, is provided. When the operating member (1365) is operated by a user, the pressing protrusion (1367) of the operating member (1365) may contact the bracket portion (1514) to push the stopper member (1500). The supporting portion (1516) is placed at the edge of the stopper member opening (1512). The supporting portion (1516) may protrude from the stopper member body (1510) and support the shuttle protrusion (1444) of the shuttle (1441).

As shown in FIG. 34, when the stopper member (1500) is located in the third position, the supporting portion (1516) supports the shuttle protrusion (1444), and may restrict the movement of the shuttle (1441) placed in the first position.

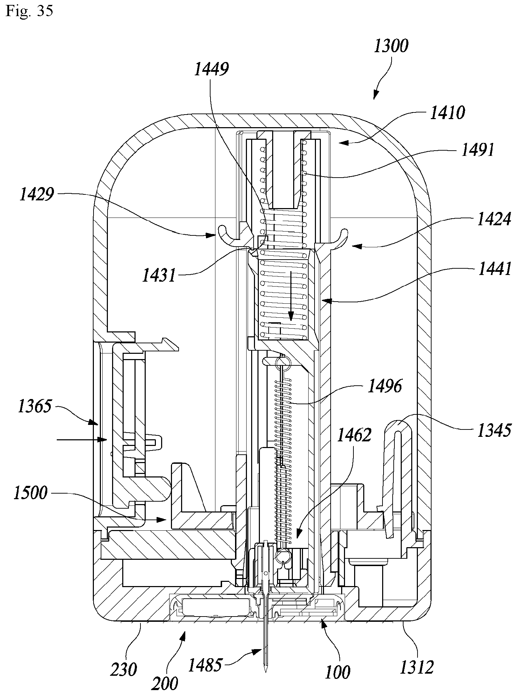

Meanwhile, as shown in FIG. 35, when a user presses the operating member (1365), the stopper member (1500) moves to the fourth position while elastically deforming the return member (1345). At this time, as the supporting portion (1516) deviates from the shuttle protrusion (1444), the shuttle (1441) moves to the second position and the needle (1485) and the sensor of the sensor unit (100) are inserted into the skin of a user. Afterwards, the needle (1485) is pulled out from the skin by the needle release driver (1495). After the shuttle (1441) moves to the second position, the detent portion (1449) of the shuttle (1441) faces the blocking portion detent (1431) of the blocking portion (1429), and the shuttle (1441) cannot be returned to the first position.

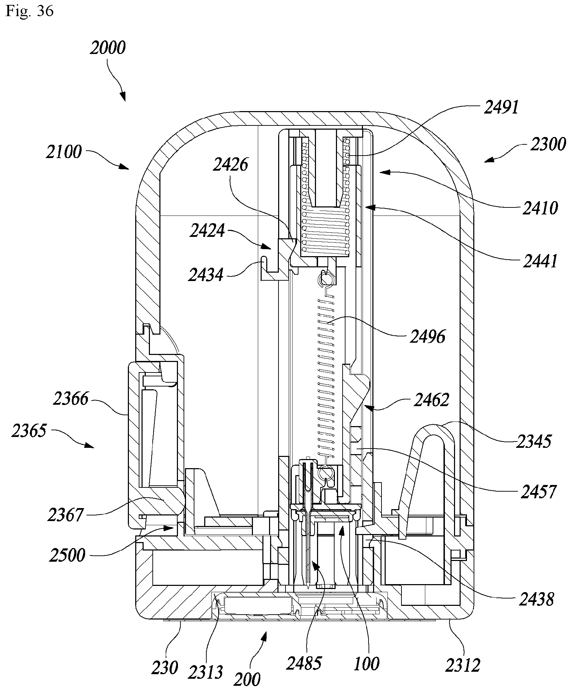

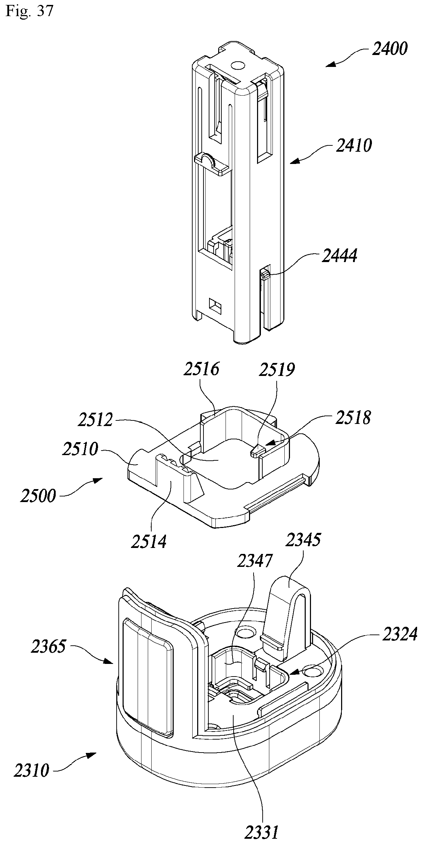

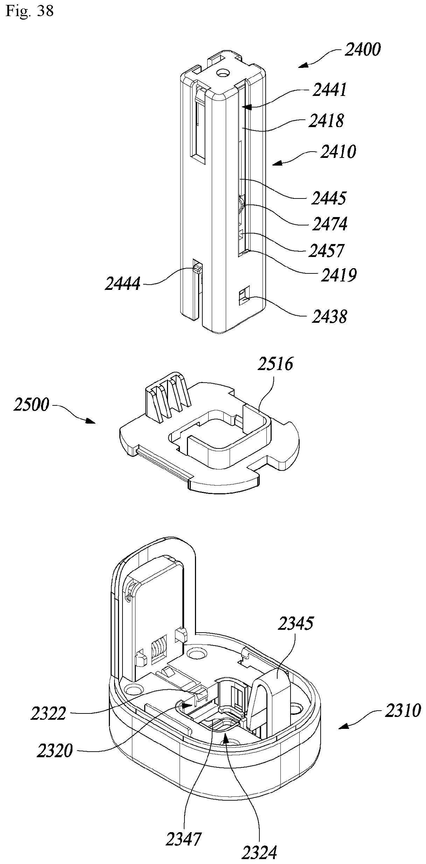

Meanwhile, FIG. 36 is a cross-sectional view showing an applicator assembly according to another embodiment of the present disclosure, and FIGS. 37 and 38 are exploded perspective views showing a portion of the applicator assembly shown in FIG. 36.

The applicator assembly (2000) according to another embodiment of the present disclosure includes a sensor unit (100) including a sensor (110) that is inserted into the skin of a user and measures biometric information, a base unit (200) constituting a body attachable unit with the sensor unit (100), and an applicator (2100) for attaching the body attachable unit to the skin of a user. The sensor unit (100) and the base unit (200) may be installed on the applicator (2100) in a separated state, and form a body attachable unit by being coupled to each other by the applicator (2100).

The applicator (2100) includes an applicator body (2300) to which the base unit (200) is detachably coupled, an operating member (2365) installed on the applicator body (2300) for user manipulation, and as insertion unit assembly (2400) including an insertion unit (2440) for inserting the sensor (110) into the skin of a user, and a stopper member (2500) movably installed on the applicator body (2300).

The applicator body (2300) includes a base frame (2310) and a top case (2360) that covers the upper part of the base frame (2310).

The base frame (2310) has a bottom portion (2312) that can be in contact with the skin of a user. The bottom portion (2312) of the base frame (2310) is provided with a recess (2313) on which the base unit (200) is mounted. A stage (2331) supporting the stopper member (2500) is provided on the upper part of the base frame (2310). An applicator body groove (2347) into which the insertion unit assembly (2400) is inserted is provided in the middle of the stage (2331). An applicator body snap portion (2320) is provided inside the applicator body groove (2347) to secure the column member (2410) of the insertion unit assembly (2400). The applicator body snap portion (2320) includes a snap hook (2322) that engages the column member (2410) of the insertion unit assembly (2400). The applicator body snap portion (2320) forms an applicator body coupling portion (2324) for fixing the column member (2410) to the applicator body (2300) with the applicator body groove (2347).

A return member (2345) capable of returning the stopper member (2500) to its original position is installed on one side of the base frame (2310). The return member (2345) can be elastically deformed by the stopper member (2500) and can return the stopper member (2500) moved by the operating member (2365) to its original state. The return member (2345) is elastically deformed by the stopper member (2500) advanced by the operating member (2365) and can apply an elastic force to the stopper member (2500) in a retreating direction.

The operating member (2365) includes a button portion (2366) and a pressing protrusion (2367) protruding from the button portion (2366). When the operating member (2365) is pressed into the applicator body (2300) by a user, the pressure protrusion (2367) pushes the stopper member (2500) toward the return member (2345), thereby moving the stopper member (2500).

The insertion unit assembly (2400) includes a column member (2410) fixed to the applicator body (2300), and an insertion unit (2440) supported by the column member (2410) to be coupled to the sensor unit (100) and moves the sensor unit (100) from the first position to the second position. The insertion unit (2440) is coupled to the applicator body (2300) through the column member (2410). The insertion unit (2440) includes a shuttle (2441) movably installed inside the column member (2410), and a needle assembly (2460) that can move together with the shuttle (2441). The shuttle (2441) is coupled with the sensor unit (100) and can move from the first position to the second position inside the applicator body (2300). The needle assembly (2460) includes a carrier (2462) that can relatively move with respect to the shuttle (2441) and a needle (2485) that is coupled to the carrier (2462) and can be inserted into the skin of a user.

The column member (2410) supports the insertion unit (2440) by being fixed to the applicator body (2300). The column member (2410) includes a column member body (2411). The column member body (2411) has a space inside to accommodate the insertion unit (2440), and has a column member opening (2412) open to the outside at one end. One end of the column member body (2411) is provided with a coupling end portion (2437) that is inserted into the applicator body groove (2347) of the applicator body (2300). Additionally, one end of the column member body (2411) is provided with a column member snap portion (2414) that can engage with the applicator body snap portion (2320) of the applicator body (2300). The column member snap portion (2414), together with the coupling end portion (2437), constitutes a column member coupling portion (2436) coupled to the applicator body coupling portion (2343). The column member (2410) can be more firmly fixed to the applicator body (2300) by engaging the column member snap portion (2414) with the applicator body snap portion (2320). The column member snap portion (2414) includes a snap groove (2415) into which the snap hook (2322) of the applicator body snap portion (2320) is inserted.

The column member (2410) can be simply coupled to the applicator body (2300) by inserting one end into the applicator body groove (2347) of the applicator body (2300).

A slit (2418) is formed on the side of the column member body (2411) in a direction parallel to the moving direction of the shuttle (2441). A release portion (2419) is provided in the middle part of the slit (2418). The release portion (2419) is a part of the carrier (2462) that can be contacted while the carrier (2462) moves toward the base unit (200). Due to the action of the release portion (2419), the carrier (2462) may relatively move with respect to the shuttle (2441) by being disengaged from the shuttle (2441).

Additionally, guide grooves (2420) are formed on both sides of the column member body (2411) in a direction parallel to the moving direction of the shuttle (2441). The shuttle hook (2443) of the shuttle (2441) is positioned in the guide groove (2420), so that the shuttle hook (2443) can move along the guide groove (2420). A column member detent (2421) is provided at the end of the guide groove (2420) to limit the moving distance of the shuttle (2441). When the shuttle (2441) moves from the first position with the sensor unit (100) and reaches the second position, the shuttle hook (2443) comes in contact with the column member detent (2421) so that the shuttle (2441) can stop at the second position.

A clamping portion (2424) and a blocking portion (2429) that can restrict the movement of the shuttle (2441) are provided on another side of the column member body (2411).

The clamping portion (2424) may engage with the shuttle (2441) and may be used to temporarily fix the shuttle (2441) to the column member (2410) during the process of assembling the insertion unit assembly (2400). The clamping portion (2424) includes a clamping portion detent (2426) that can be engaged with one side of the shuttle (2441). The clamping portion detent (2426) may be formed to protrude inward from the column member body (2411), or may be formed to engage with one side of the shuttle (2441).

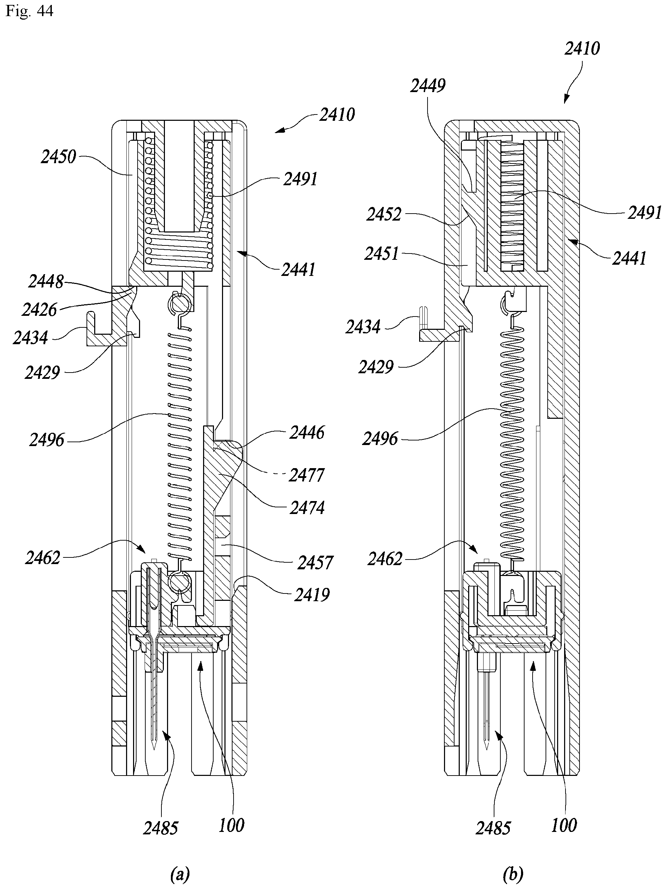

The insertion unit assembly (2400) may be coupled to the applicator body (2300) in a state in which the shuttle (2441) is temporarily fixed to the column member (2410) by the clamping portion (2424). While the shuttle (2441) is temporarily fixed to the column member (2410) by the clamping portion (2424), the shuttle (2441) cannot move. As shown in (a) of FIG. 44, after the shuttle (2441) is inserted into the column member (2410), when the clamping portion detent (2426) of the clamping portion (2424) is engaged with to the shuttle latch (2448) of the shuttle (2441), the shuttle (2441) is temporarily fixed to the column member (2410). Since the shuttle latch (2448) is engaged with the clamping portion detent (2426), the shuttle (2441) cannot relatively move with respect to the column member (2410). After the insertion unit assembly (2400) is coupled to the applicator body (2300), the insertion unit (2440) may operate after the clamping portion detent (2426) is disengaged from the shuttle latch (2448).

The blocking portion (2429) is for fixing the shuttle (2441), which has reached the second position, at the second position. The blocking portion (2429) includes a blocking portion detent (2429) that can be in contact with one side of the shuttle (2441). The blocking portion detent (2429) may have a shape that protrudes inward from the column member body (2411), or may have another shape that can be in contact with one side of the shuttle (2441).

After the applicator (2100) operates to insert the sensor (110) into the skin of a user, the applicator (2100) needs to be separated from the body attachable unit and disposed of. The blocking portion (2429) may prevent reuse of the applicator (2100) by restricting the movement of the shuttle (2441) after the shuttle (2441) moves to the second position. After the shuttle (2441) moves to the second position, the blocking portion detent (2429) comes into contact with one side of the shuttle (2441), thereby preventing the shuttle (2441) from moving to the first position.

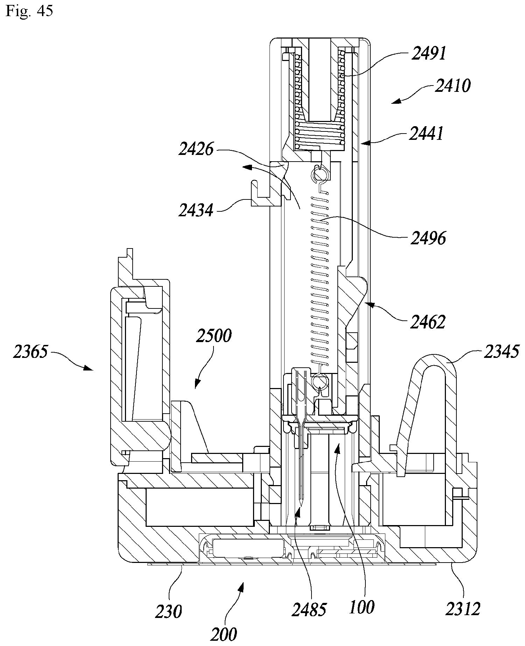

The clamping portion (2424) and the blocking portion (2428) are provided on the column member arm (2431) which is elastically deformably connected to the column member body (2411). The column member arm (2431) is provided with a knob (2434) for user operation. When a user pulls the knob (2434), the clamping arm (2425) may be elastically deformed. After coupling the insertion unit assembly (2400) to the applicator body (2300) as shown in FIG. 45, a user pulls the knob (2434) to elastically deform the column member arm (2431), thereby releasing the clamping portion detent (2426) from the shuttle latch (2448).

The shuttle (244)1 is installed to be linearly movable inside the column member (2410). The shuttle (2441) includes a shuttle body (2442) with a space inside to accommodate the needle assembly (2460). A shuttle hook (2443) is provided on one side of the shuttle body (2442) to limit the movement range of the shuttle (2441). A shuttle protrusion (2444) is provided on the other side of the shuttle (2441). The shuttle protrusion (2444) protrudes from the shuttle body (2442) so as to contact the stopper member (2500). The shuttle (2441) is fixed at the first position when the shuttle protrusion (2444) comes into contact with the stopper member (2500), and can move to the second position when the shuttle protrusion (2444) leaves the stopper member (2500).

A slit (2445) is formed on the side of the shuttle body (2442) in a direction parallel to the moving direction of the shuttle (2441). The middle portion of the slit (2445) is provided with a shuttle detent (2446) with which the carrier latch (2477) of the carrier (2462) can engage. In a state in which the carrier latch (2477) is engaged with the shuttle detent (2446), the carrier (2462) moves with the shuttle (2441) and cannot relatively move with respect to the shuttle (2441). And, when the carrier latch (2477) is disengaged from the shuttle detent (2446), the carrier (2462) can relatively move with respect to the shuttle (2441).

On both sides of the shuttle body (2442), a shuttle latch (2448) that can engage with the clamping portion (2424) of the column member (2410), and a detent portion (2449) that can contact the blocking portion (2429) are provided.

When the shuttle latch (2448) engages with the clamping portion detent (2426) of the clamping portion (2424), the shuttle (2441) may be temporarily fixed to the column member (2410). When the temporary fixing state by the clamping portion (2424) is released, the clamping portion detent (2426) is inserted into the guide groove (2450) formed in the shuttle body (2442) to be adjacent to the shuttle latch (2448). The guide groove (2450) is formed in a direction parallel to the moving direction of the shuttle (2441). When the shuttle (2441) moves from the first position to the second position, the clamping portion detent (2426) may relatively move with respect to the shuttle (2441) in the guide groove (2450). The shape or location of the shuttle latch (2448) is not limited to that shown and may be changed in various ways.

The detent portion (2449) is provided at the end of the shuttle body (2442) so as to contact the blocking portion detent (2429) of the blocking portion (2429). After the shuttle (2441) moves to the second position, the detent portion (2449) is placed to face the blocking portion detent (2429), so that the shuttle (2441) cannot move to the first position. The blocking portion detent (2429) is inserted into the guide groove (2451) formed in the shuttle body (2442) and can relatively move with respect to the shuttle (2441). The guide groove (2451) is formed in a direction parallel to the moving direction of the shuttle (2441). A shuttle inclined portion (2452) is positioned on one side of the guide groove (2451). The shuttle inclined portion (2452) is provided in an inclined form with respect to the moving direction of the shuttle (2441). When the shuttle (2441) moves to the second position, the blocking portion detent (2429) relatively move with respect to the guide groove (2451), and when it reaches the shuttle inclined portion (2452), the column member arm (2431) may be elastically deformed. As the column member arm (2431) is elastically deformed, the blocking portion detent (2429) can smoothly pass through the shuttle inclined portion (2452) and reach a position facing the detent portion (2449). The shape or location of the detent portion (2449) is not limited to that shown and may be changed in various ways.

A shuttle bottom portion (2453) in contact with the sensor unit (100) is provided at one end of the shuttle body (2442). The shuttle bottom portion (2453) is provided with a shuttle through-hole (2454) into which the needle (2485) is inserted, and a pressing protrusion (2455) inserted into the sensor unit groove (138) of the sensor unit (100).

In addition, the shuttle (2441) includes a shuttle locking portion (2456) and a shuttle opening (2458). The shuttle locking portion (2456) includes a locking groove (2457) into which the locking protrusion (525) of the stopper member (500) can be inserted. When the shuttle (2441) reaches the second position inside the column member (2410), the locking groove (2457) faces the opening (2438) of the column member (2410). Shuttle opening (2458) is formed on one side of the shuttle body (2442) to open outward. During the process of assembling the insertion unit assembly (2400), the needle assembly (2460) may be installed inside the shuttle body (2442) through the shuttle opening (2458).

The shuttle (2441) moves by receiving movement force from the shuttle driver (2490). The shuttle driver (2490) includes an elastic member (2491) that applies elastic force to the shuttle (2441) in the direction of moving from the first position to the second position. As shown in (a) of FIG. 44, the shuttle (2441) may remain temporarily fixed at the column member (2410) in a state in which the elastic member (2491) is elastically deformed before the column member (2410) is coupled to the applicator body (2300). And, after the insertion unit assembly (2400) is coupled to the applicator body (2300), when the clamping portion (2424) is disengaged from the shuttle latch (2448), the shuttle protrusion (2444) comes into contact with the stopper member (2500) and the shuttle (2441) may remain stopped in the first position.

The needle assembly (2460) includes a carrier (2462) to which the sensor unit (100) is detachably coupled, and a needle (2485) coupled to the carrier (2462) to penetrate the sensor unit (100). The needle (2485) includes a needle body (2486) that can be inserted into the skin of a user, and a needle head (2487) coupled to the carrier (2462).

The carrier (2462) includes a carrier body (2463) to which the needle (2485) is coupled, an elastically deformable carrier wing (2471) extending from the carrier body (2463), and a grip arm (2479) connected to the side of the carrier body (2463). An insertion groove (2465) into which a portion of the needle (2485) is inserted is formed on one side of the carrier body (2463). The carrier wing (2471) is elastically deformably connected to the carrier body (2463). Carrier wing (2471) includes trigger (2474) and carrier latch (2477). The trigger (2474) may be inserted into the slit (2445) of the shuttle (2441) and the slit (2418) of the column member (2410), and may contact the release portion (2419) of the column member (2410). Carrier latch (2477) may engage shuttle detent (2446) of shuttle (2441). In a state in which the carrier latch (2477) is engaged with the shuttle detent (2446), the carrier (2462) can maintain the elastic member (2496) in an elastically deformed state and cannot move in the direction in which the elastic force of the elastic member (2496) acts. And, when the trigger (2474) contacts the release portion (2419) and the carrier wing (2471) is elastically deformed, the carrier latch (2477) may deviate from the shuttle detent (2446) and the carrier (2462) can move. The grip arm (2479) is provided to be elastically deformable on the side of the carrier body (2463) and can engage with the sensor unit (100). The grip arm (2479) is provided with a grip protrusion (2480) engaged with the sensor unit housing (220) and a protrusion (2481) in contact with the inner wall of the column member (2410).

The carrier (2462) is coupled to the shuttle (2441) so that the carrier body (2463) is located inside the shuttle (2441) and the end of the grip arm (2479) protrudes from the shuttle (2441). And the carrier (2462) can fix the sensor unit (100) to the shuttle (2441) in such a way that a pair of grip arms (2479) engage with the sensor unit (100).

The carrier (2462) can be relatively moved with respect to shuttle (2441) by needle release driver (2495). The needle release driver (2495) includes an elastic member (2496) that provides a moving force to the carrier (2462).

As shown in FIGS. 44 and 45, the insertion unit assembly (2400) is formed by inserting the applicator body (2300) in a state in which the elastic member (2491) of the shuttle driver (2490) and the elastic member (2496) of the needle release driver (2495) are elastically deformed can be coupled. Additionally, the insertion unit assembly (2400) may be coupled to the applicator body (2300) in a state being coupled to the sensor unit (100). As described above, in the process of coupling the insertion unit assembly (2400) to the applicator body (2300), the shuttle (2441) may be temporarily fixed to the column member (2410) by the clamping portion (2424), so that accidental movement of the shuttle (2441) can be prevented.

As shown in FIG. 45, after the insertion unit assembly (2400) is coupled to the applicator body (2300), the clamping portion (2424) is manipulated to release the restraining force on the shuttle (2441), so that the insertion unit (2440) may be brought into an operable state by the operating member (2365). For example, when a user pulls the knob (2434) to elastically deform the column member arm (2431), the clamping portion detent (2426) may be released from the shuttle latch (2448).

When the clamping portion detent (2426) is released from the shuttle latch (2448), the shuttle (2441) is slightly pushed by the elastic member (2491) of the shuttle driver (2490), so that the clamping portion detent (2426) is located in the guide groove (2450) of the shuttle (2441) and can be maintained out of the shuttle latch (2448). At this time, the shuttle protrusion (2444) of the shuttle (2441) comes into contact with the stopper member (2500), and the shuttle (2441) can remain stopped in the first position in a state in which the elastic member (2491) is elastically deformed.

As such, the applicator assembly (2000) according to this embodiment of the present disclosure can be simply assembled to the applicator body (1300) by forming a single assembly in which the shuttle (2441), a needle assembly (2460), a shuttle driver (2490), and a needle release driver (2495), which constitute the insertion unit (2440), together with the column member (2410). Therefore, installation of the insertion unit (2440) is easy, and manufacturing time can be reduced.

The stopper member (2500) is positioned to move linearly on the stage (2331) of the middle frame (2330). The stopper member (2500) may move from a third position, which supports the shuttle (2441) to be positioned in the first position, to a fourth position where it is disengaged from the shuttle (2441). The stopper member (2500) is in contact with the shuttle protrusion (2444) of the shuttle (2441) located in the first position at the third position to restrict the movement of the shuttle (2441) placed in the first position, and when moving to the fourth position, the shuttle (2441) can be released from the shuttle protrusion (2444) to move to a second position.

The stopper member (2500) includes a stopper member body (2510), a supporting portion (2516) for supporting the shuttle (2441), and a stopper member locking portion (2518) for restraining the movement of the shuttle (2441) moved to the second position. The stopper member locking portion (2518) includes a locking protrusion (2519) inserted into the locking groove (2457) of the shuttle (2441).

A stopper member opening (2512) into which the insertion unit assembly (2400) is inserted is provided in the middle of the stopper member body (2510). At one end of the stopper member body (2510), a bracket portion (2514) that can be contacted by the operating member (2365) is provided.

As shown in FIG. 36, when the stopper member (2500) is located in the third position, the supporting portion (2516) supports the shuttle protrusion (2444) to restrict the movement of the shuttle (2441) located in the first position.

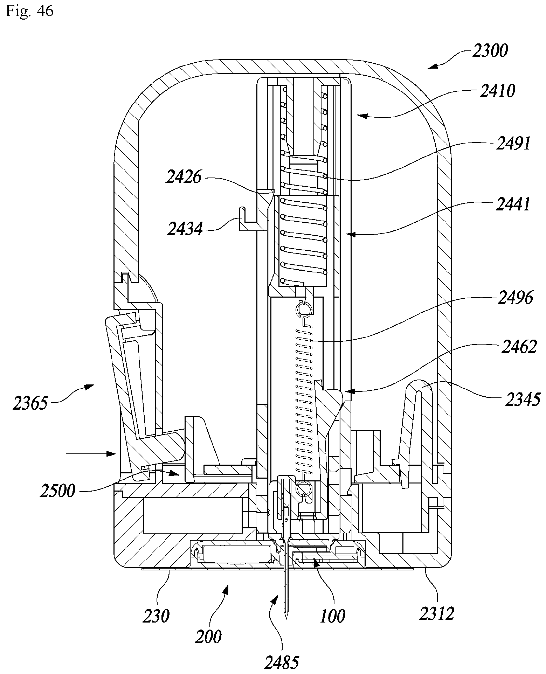

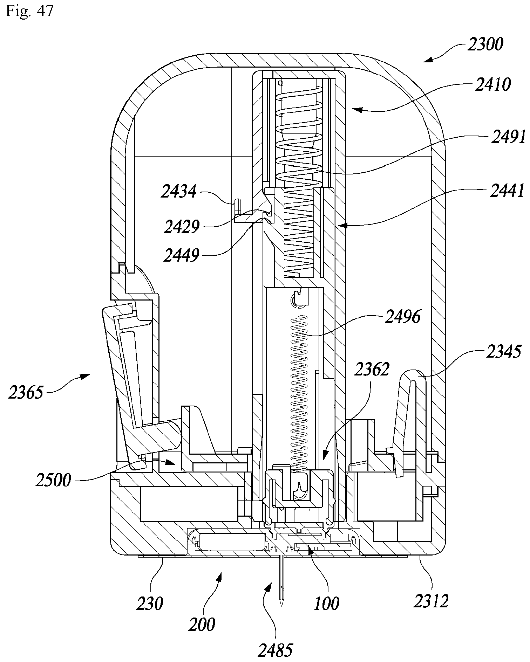

Meanwhile, as shown in FIGS. 46 and 47, when a user presses the operating member (2365), the stopper member (2500) moves to the fourth position while elastically deforming the return member (2345). At this time, as the supporting portion (2516) deviates from the shuttle protrusion (2444), the shuttle (2441) moves to the second position, and the needle (2485) and the sensor of the sensor unit (100) are inserted into the user's skin. Afterwards, the needle (2485) is pulled out from the skin by the needle release driver (2495). After the shuttle (2441) moves to the second position, the detent portion (2449) of the shuttle (2441) faces the blocking portion detent (2429) of the blocking portion (2428), and the shuttle (2441) cannot be returned to the first position.

Additionally, when the force applied to the operating member (2365) by a user is removed, the stopper member (2500) returns to the third position by the elastic force of the return member (2345). At this time, the locking protrusion (2519) of the stopper member (2500) passes through the opening (2438) of the column member (2410) and is inserted into the locking groove (2457) of the shuttle (2441) that has moved to the second position. Accordingly, the movement of the shuttle (2441) that has moved to the second position is restricted.

Although the present disclosure has been shown and described in connection with preferred embodiments for illustrating the principles of the disclosure, the disclosure is not limited to the construction and operation as shown and described. Rather, those skilled in the art will understand that numerous changes and modifications can be made to the present disclosure without departing from the concept and scope of the attached registration claims.

## Claims

1. An applicator for inserting a sensor for measuring biometric information into skin of a user, the applicator comprising:
an applicator body having an applicator body coupling portion;
an insertion unit to move a sensor unit, including the sensor and a sensor unit housing to which the sensor is mounted, from a first position spaced apart from the skin of the user to a second position where the sensor is inserted into the skin of the user; and
a column member which the insertion unit is mounted to, has a column member coupling portion corresponding to the applicator body coupling portion, and is fixed to the applicator body in a way that the column member coupling portion is engaged with the applicator body coupling portion.

2. The applicator according to claim 1, wherein:
the applicator body coupling portion includes an applicator body groove formed at one side of the applicator body,
the column member includes a column member body having a space, in which the insertion unit is mounted, inside the column member body, and
the column member coupling portion includes a coupling end portion provided at one end of the column member body to be inserted into the applicator body groove.

3. The applicator according to claim 2,
wherein the applicator body coupling portion includes:
a fence portion configured to be elastically deformable in the applicator body groove to support the coupling end portion by being contacted with an outer surface of the coupling end portion.

4. The applicator according to claim 2,
wherein the coupling end portion includes:
a plurality of column member legs configured to be elastically deformable at one end of the column member body to contactable with the applicator body.

5. The applicator according to claim 1,
wherein one of the applicator body coupling portion and the column member coupling portion has an elastically deformable snap hook, and another of the applicator body coupling portion and the column member coupling portion has a snap groove into which a portion of the snap hook is inserted.

6. The applicator according to claim 1,
wherein the insertion unit comprises:
a shuttle configured to be movable from the first position to the second position together with the sensor unit, and
an elastic member configured to apply an elastic force to the shuttle in a direction of moving toward the second position with respect to the shuttle, and
wherein the column member is configured to guide the shuttle to move linearly the shuttle.

7. The applicator according to claim 6,
wherein the shuttle is temporarily fixed to the column member such that the column member maintains a position in which the elastic member is elastically deformed by the clamping portion, which connects the shuttle and the column member, before the column member is coupled to the applicator body, and after the column member is coupled to the applicator body, a temporarily fixed state by the clamping portion is released.

8. The applicator according to claim 7,
wherein the clamping portion includes a clamping hook elastically deformably connected to the shuttle to be engaged with a column member detent provided at one side of the column member, and
wherein the shuttle is configured to maintain a position in which the elastic member is elastically deformed when the clamping hook is engaged with the column member detent, and the temporarily fixed state is released when the clamping hook is disengaged from the column member detent.

9. The applicator according to claim 7,
wherein the clamping portion includes a clamping portion arm provided to be elastically deformable at the column member to be engaged with a shuttle latch provided at one side of the shuttle,
wherein the shuttle is configured to maintain a position in which the elastic member is elastically deformed when the clamping portion arm is engaged with the shuttle latch, and the temporarily fixed state is released when the clamping portion arm is disengaged from the shuttle latch.

10. The applicator according to claim 6,
wherein the column member is provided with a blocking portion configured to restrict movement of the shuttle to prevent the shuttle from returning to the first position by being contacted with one side of the shuttle after the shuttle moves to the second position.

11. The applicator according to claim 1,
wherein the insertion unit includes:
a shuttle configured to move from the first position to the second position together with the sensor unit, and
an elastic member configured to apply elastic force to the shuttle in a direction of moving to the second position with respect to the shuttle,
wherein the column member is configured to guide the shuttle to move linearly the shuttle, and
wherein the shuttle is temporarily fixed to the column member to maintain a position of elastically deforming the elastic member by a safety pin that passes through the column member hole formed at one side of the column member and is inserted into the shuttle hole provided at one side of the shuttle before the column member is coupled to the applicator body, and, after the column member is coupled to the applicator body, a state temporarily fixed by the safety pin is released.

12. The applicator according to claim 11,
wherein the insertion unit includes:
a carrier relatively movably mounted to the shuttle and having a carrier hole into which the safety pin is insertable,
a needle coupled to the carrier to be inserted into the skin of the user together with the sensor, and
a needle release elastic member configured to apply elastic force to the carrier in a direction in which the needle is extracted from the skin of the user, and
wherein the carrier is temporarily fixed to the shuttle to maintain a position in which the needle release elastic member is elastically deformed by inserting the safety pin into the carrier hole before the column member is coupled to the applicator body, and after the column member is coupled to the applicator body, the state temporarily fixed by the safety pin is released.

13. An applicator assembly comprising:
an applicator body having an applicator body coupling portion;
a sensor unit including a sensor and a sensor unit housing to which the sensor is mounted;
an insertion unit to move the sensor unit from a first position spaced apart from the skin of the user to a second position where the sensor is inserted into the skin of the user; and
a column member which the insertion unit is mounted to, has a column member coupling portion corresponding to the applicator body coupling portion, and is fixed to the applicator body in a way that the column member coupling portion is engaged with the applicator body coupling portion.

14. The applicator assembly according to claim 13, comprising a base unit including a base unit housing to which the sensor unit housing is coupled and an adhesive portion provided at the base unit housing to be attached to the skin of the user, the base unit configured to be spaced apart from the sensor unit to be separably coupled to the applicator body,
wherein the sensor unit housing is coupled to the base unit housing at the second position.
